(19) Europäisches Patentamt
European Patent Office
Office européen des brevets

(11) Publication number: **0 118 875**
**B1**

(12) **EUROPEAN PATENT SPECIFICATION**

(45) Date of publication of patent specification: **16.12.87**

(21) Application number: **84102429.2**

(22) Date of filing: **07.03.84**

(51) Int. Cl.[4]: **C 07 D 499/00,** A 61 K 31/43 // C07D233/54, C07D249/08, C07D405/12

(54) **6-(Hydroxyalkyl)-2-(heteroarylalkylthio)-penems, pharmaceutical compositions containing them, and processes for their preparation.**

(30) Priority: **14.03.83 US 475281**
**22.12.83 US 564586**

(43) Date of publication of application:
**19.09.84 Bulletin 84/38**

(45) Publication of the grant of the patent:
**16.12.87 Bulletin 87/51**

(84) Designated Contracting States:
**AT BE CH DE FR GB IT LI LU NL SE**

(56) References cited:
**EP-A-0 002 210**
**EP-A-0 003 960**
**EP-A-0 013 662**

**The file contains technical information submitted after the application was filed and not included in this specification**

(73) Proprietor: **SCHERING CORPORATION**
**2000 Galloping Hill Road**
**Kenilworth, New Jersey 07033 (US)**

(72) Inventor: **Girijavallabhan, Viyyoor Moopil**
**10 Maplewood Drive**
**Parsippany, NJ 07054 (US)**
Inventor: **Ganguly, Ashit Kumar**
**96 Cooper Avenue**
**Upper Montclair, NJ 07043 (US)**
Inventor: **Pinto, Patrick Anthony**
**232 Randolph Avenue**
**Mine Hill, NJ 07801 (US)**
Inventor: **Versace, Richard William**
**13 Edgewood Road**
**Ringwood, NJ 07456 (US)**

(74) Representative: **Ritter, Stephen David et al**
**Mathys & Squire 10 Fleet Street**
**London EC4Y 1AY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 118 875 B1

**Description**

This invention relates to novel 6-(hydroxyalkyl)-2-(heteroarylalkylthio)penems having the general formula:

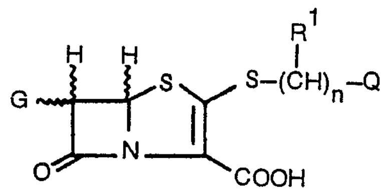

I

wherein

G is hydroxyloweralkyl,
Q is the group

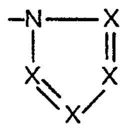

wherein

each X is $=\overset{R}{\overset{|}{C}}-$ or $=N-$, with the proviso that at least one X is $=\underset{R}{\underset{|}{C}}-$;

R is hydrogen, loweralkyl, aminoloweralkyl, mono- and di-loweralkylaminoloweralkyl, carboxyloweralkyl, sulfoloweralkyl, hydroxyloweralkyl, cyano, hydroxy, amino, mono- and di-loweralkylamino, loweralkylsulfonate, sulfamyl, halogeno, hydroxyliminoloweralkyl, loweralkoxyiminoloweralkyl, carboxy, carbamyl, mono- or di-loweralkylcarbamyl, nitro, carbamyloxy, ureidoloweralkyl, or carbamylhydrazoloweralkyl; or two R groups attached to adjacent carbon atoms, together with the carbon atoms to which they are attached, form a fused aromatic ring;

each $R^1$ is independently hydrogen, loweralkyl, carboxy, carbamyl, cyano, hydroxy, amino, loweralkylthio, fluoro, loweralkoxy, loweralkanoyloxy, or loweralkyl substituted by imidazolyl (which may be substituted by a group defined by R or by ureido), amino, loweralkylamino, carbamoylamino, ureidoamino, hydroxy, cyano, halogen, loweralkoxy, carbamyloxy, carboxy, carbamyl, loweralkylcarbonyl, sulfo, sulfamyl, loweralkylsulfonyl, loweralkoxysulfonyl, loweralkoxycarbonyl, hydroxyloweralkylcarbonyl or hydroxyloweralkylsulfonyl, with the proviso that when $R_1$ is attached to a carbon atom adjacent either to the sulphur atom connected to the 2-position of the penem ring or the nitrogen atom by which the group Q is connected to the side chain

$$-\overset{R^1}{\overset{|}{(CH)_n}}-$$

then such $R_1$ cannot be hydroxy, amino or fluoro;

n is 1 to 4; and

the pharmaceutically acceptable salts and esters thereof, in racemic or optically active form.

Preferred compounds of formula I are those wherein the group Q is chosen from the following groups which may be unsubstituted or substituted; pyrrolyl, imidazolyl, pyrazolyl, 1,2,4-triazolyl, 4,1,2-triazolyl, 1,2,3-triazolyl, 2,1,3-triazolyl, 1,2,3,4-tetrazolyl and 2,1,3,4-tetrazolyl. The substituents, if present, are 1 to 4 "R" groups. Most preferred are compounds of formula I wherein the group Q is imidazolyl or a triazolyl group.

A preferred fused aromatic ring which may be contained in Q is a six-membered aromatic ring which may contain up to 3 nitrogen atoms as ring heteroatoms. Typical examples of groups Q containing such a fused aromatic ring are indolyl, isoindolyl, isoindazolyl, benzimidazolyl, indazolyl, 1,2,3-benzotriazolyl, 2,1,3-benzotriazolyl, 4,5,6 or 7-azaindolyl, 4,5,6 or 7-azabenzimidazolyl, 4,5,6 or 7-azabenzopyrazolyl, 1-pyrazolo-[3,4-d]-pyrimidinyl, 2-pyrazolo-[4,3-c]-pyridinyl, 3-v-triazolo-[4,5-b]-pyridinyl, 1-pyrazolo-[3,4-b]-pyrazinyl, 2-v-triazolo-[4,5-b]-pyrazinyl, and 1,3,4,6-benzotetrazolyl.

Preferred $R^1$ substituents are hydrogen and substituted loweralkyl.

Preferred R substituents are amino, hydroxy and loweralkyl.

Also preferred are compounds of formula I wherein n is 2 to 4; most preferred are those compounds wherein n is 2.

G, is preferably 1-hydroxyethyl.

The term "loweralkyl" as used herein means alkyl groups of 1 to 6 carbon atoms and includes methyl, ethyl, propyl, butyl, pentyl and hexyl and the corresponding branched chain isomers thereof. Similarly, "loweralkoxy" means straight or branched alkoxy groups having 1 to 6 carbon atoms, e.g., methoxy, ethoxy, propoxy and butoxy, and "loweralkanoyloxy" means straight or branched chain alkanoyloxy groups of 1 to 6 carbon atoms, e.g. acetoxy, propionoxy, and butyryloxy.

Compounds of the present invention possess a number of asymmetric carbon atoms, indicated in the partial formula II below at the 5, 6, and possibly in the 2′ to 5′-position carbon atoms. Also where G is hydroxy loweralkyl other than hydroxymethyl the carbon atom to which the hydroxy group is attached will be asymmetric.

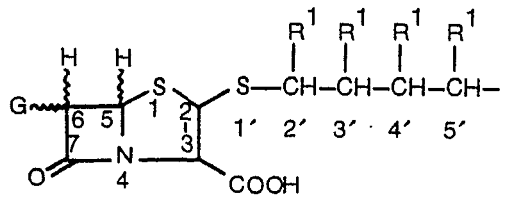

II

Where G is 1-hydroxyethyl the compounds of the invention preferably possess 5R,6S,8R or 5R,6R,8S stereochemistry at the indicated chiral atoms. The preferred absolute stereochemistry for such compounds of the present invention at those positions is 5R,6S,8R.

Compounds of formula I wherein $R^1$ is other than hydrogen will have asymmetric carbon atom(s) as shown in formula II at the 2′ to 5′ positions.

The present invention contemplates compounds of formula I in stereospecific form or as mixtures of such stereoisomers.

As used herein, "pharmaceutically acceptable salts" preferably means alkali metal salts such as sodium and potassium salts; alkaline earth metal salts such as calcium, magnesium and aluminum salts; amine salts formed from a wide variety of suitable organic amines, i.e., aliphatic, cycloaliphatic, (cycloaliphatic)aliphatic or araliphatic primary, secondary or tertiary mono-, di- or polyamines, or heterocyclic bases, e.g., salts derived from triethylamine, 2-hydroxyethylamine, di-(2-hydroxyethyl)amine, tri-(2-hydroxyethyl)amine, 4-aminobenzoic acid-2-diethylaminoethyl ester, 1-ethylpiperidine, bicyclohexylamine, N,N′-dibenzylethylenediamine, pyridine, collidine, quinoline, procaine, dibenzylamine, 1-ephenamine and N-alkylpiperidine; or acid addition salts formed from mineral acids such as hydrochloric, hydrobromic, hydroiodic, phosphoric or sulfuric acids, or formed from organic carboxylic or sulfonic acids such as trifluoroacetic, para-toluene sulfonic, maleic, acetic, citric, oxalic, succinic, benzoic, tartaric, fumaric, mandelic, ascorbic and malic acids.

Compounds of this invention which contain a 3-carboxylic group and a basic group (which may be an $R^1$ group e.g. an amine), or the group Q (especially a group Q containing two nitrogen atoms) may form an inner salt i.e. a zwitterion.

"Pharmaceutically acceptable esters" means physiologically cleavable esters, i.e., metabolizable esters known in the penicillin, cephalosporin and penem arts to be easily cleaved within the body to the parent acid. Examples of groups forming such esters are indanyl, phthalidyl, methoxymethyl, glycyloxymethyl, phenylglycyloxymethyl, thienylglycyloxymethyl, acetoxymethyl and pivaloyloxymethyl.

When tested in standardized microbiological assay, the compounds of this invention are active against such gram-positive organisms as *Staphylococcus epidermis* and *Bacillus subtilis,* and such gram-negative organisms as *E. coli* and *Salmonella,* at test levels of 0.01 to 1.0 micrograms/ml. Additionally, they show activity against organisms which produce beta-lactamases, e.g. penicillinase and cephalosperinase, indicating a stability toward these enzymes. For instance, 5R,6S,8R-2-[2-(imidazol-1-yl)-ethylthio]-6-(1-hydroxyethyl)penem-3-carboxylic acid is active against *Enterobacter* 72012502 at a test level of 0.250 microgram/ml. When tested against *E. coli* 74081501 TEM—1 (a beta-lactamase producing organism) the compound exhibits activity at 0.031 microgram/ml. When tested against over sixty organisms, the mean test level against gram-negative organisms was 0.135 microgram/ml and against gram-positive organisms was 0.061 microgram/ml.

The compounds of this invention and their metabolites have little or no unpleasant odour.

As antibacterial agents, the compounds of this invention are conveniently formulated for oral, parenteral, topical and transdermal use. Thus, this invention includes within its scope pharmaceutical compositions comprising the compounds of this invention in admixture with a pharmaceutically acceptable carrier therefor. In the foregoing compositions, the compounds of this invention can be used as the sole active antibacterial agent or in combination with other antibacterial agents and/or enzyme inhibitors.

For oral administration, the compounds of this invention are typically formulated in the form of tablets, capsules, elixirs, or the like. For parenteral administration they may be formulated into solutions or suspensions.

The dosage of a compound of this invention which is administered will be dependent on the judgment of the attending clinician taking into account a variety of factors, i.e., the age and weight of the individual

being treated, the mode of administration, and the type and severity of the bacterial infection being prevented or reduced and the potency of the specific compound administered. Typically, the dosage administered per day will be in the range of from about 1 to 250 mg/kg and preferably from about 5 to 20 mg/kg in divided dosages.

Typically, the dosage will be administered in dosage units containing convenient amounts, for example, 125, 250 or 500 mg of active ingredients combined with a suitable physiologically acceptable carrier or diluent.

According to another aspect of the present invention a process for preparing a compound of formula I as defined above or its salts or esters, is characterized in that:

A) a compound of formula [IX(a), IX(b)]

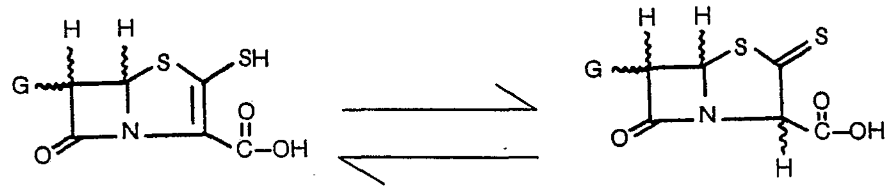

IX(a) IX(b)

in which G is as defined above, is transformed into a compound of formula I by introducing the group

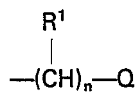

(in which Q, *n* and R[1] are as previously defined) by known conventional methods;

B) Reaction of a compound of the general formula XI

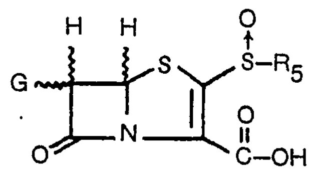

XI

in which G is as defined above and R is an organic group, with a compound of general formula XII

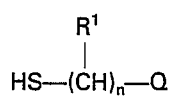

XII

in which Q, *n* and R[1] are as previously defined, or with a reactive derivative thereof, wherein the group R is different from the group

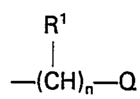

by which it is replaced,

C) intramolecular cyclisation of a compound having the general formula XIII

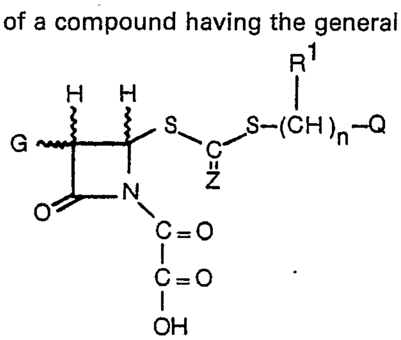

XIII

in which G, n, R[1] and Q are as defined above and Z is oxygen or sulphur, in the presence of a trivalent organophosphorous compound;

D) for the preparation of a compound of formula I in which at least one R[1] is cyano or fluoro, converting at least one hydroxyl group representing R[1] in an compound of formula I into the cyano or fluoro group;

wherein in the processes A, B, C or D, any functional groups are protected if necessary or desired, the

process A, B, C or D, being followed by removal of any protecting groups, before or after any desired separation of a stereoisomer, and isolation of the resulting penem compound of formula I as the free acid, pharmaceutically acceptable salt or pharmaceutically acceptable ester.

Process A):

The transformation of tautomer [IX(a), IX(b)] to a compound of formula I is preferably carried out using a process similar to one or other of the transformation processes described in European Patent Application No. 83111615.7, that is by:

(i) reaction with a compound of formula X

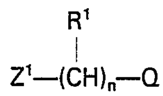

X

in which Q, $R^1$ and n are as defined above and $Z^1$ is a group leaving under the conditions of the reaction, e.g. activated hydroxy halogen, $CH_3SO_3—O—$, $O^-—P^+(C_6H_5)_3$, or the group $CF_3SO_3—$. Suitable halogens are chlorine, bromine and iodine.

Such reaction is preferably carried out in an organic solvent such as tetrahydrofuran in an inert atmosphere at a temperature typically between −5°C and 30°C. The reaction will generally be carried out in the presence of a base or acid acceptor, e.g. an inorganic carbonate, and the reaction will be generally complete within 1 to 3 hours.

(ii) Reaction with a 1,2-epoxy compound of the formula X′

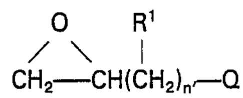

X′

in which Q and $R^1$ are as defined above and n′ is 0 to 2.

This reaction can be used for producing compounds of formula I in which $R^1$ group attached to the second carbon atom from the sulphur atom connected to the 2-position of the penem ring is hydroxy, i.e. compounds having the partial structure

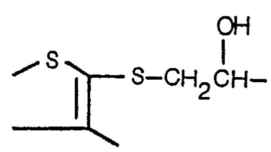

Such reaction can be carried out by combining the tautomer and the compound of formula X′ at, or about at room temperature, usually in a suitable inert solvent such as dimethylformamide.

Procedure B) involving sulfoxide replacement can be carried out, usually in an inert solvent, e.g. dichloromethane or THF. The reaction temperature is usually in the range of 0°C to −70°C.

When the thiol compound XII is itself used, the reaction is generally carried out in the presence of a base, e.g. an organic base e.g. diisopropylethylamine or triethylamine, or an inorganic base, e.g. potassium hydroxide or sodium methoxide.

Alternatively a reactive derivative, e.g. an alkali metal salt, preferably sodium or potassium may be used.

The sulfoxides of formula XI can be obtained by treating a compound of the formula XI′.

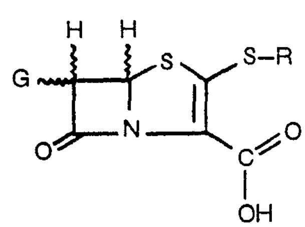

XI′

in which G and R are as defined above, with a mild oxidizing agent e.g. peroxybenzoic acid in an inert solvent, e.g. dichloromethane, at between −30°C and 20°C e.g. 0°C to 5°C.

A compound of formula XI′ can be prepared by the method disclosed in our European Patent Specification Publication No. 13662 or by the methods disclosed herein.

Procedure C), involving cyclisation of the compound of formula XIII will generally be carried out analogously to the process described in our European Patent Application, Publication No. 58317.

Thus it is usually carried out in an inert solvent, for example an aromatic hydrocarbon, e.g. toluene,

benzene, aliphatic ethers e.g. diethyl ether and dipropyl ether, cyclic ethers e.g. dioxane and tetrahydrofuran and halogenated hydrocarbons e.g. methylene chloride and chloroform.

In general the cyclization reaction is conducted at temperatures in the range from 20°C to 80°C, usually from 40°C to 60°C for a period of from 6 to 24 hours.

Suitable trivalent organophosphorous compounds are cyclic and/or acyclic trialkylphosphites, triarylphosphites and mixed arylalkylphosphites or phosphoramides. The preferred trivalent organophos'·iorous compound is a trialkylphosphite; most preferred is triethylphosphite.

Process D can be carried out by methods known in the art for replacing the hydroxy of hydroxyalkyl groups with fluoro or cyano.

A convenient general procedure is to react the hydroxy side chain with dialkylazido carboxylate (DAC) and triphenyl phosphine (TPP) to convert the hydroxy to a leaving group, which can then be displaced with fluoro or cyano anion. For example, a suitably protected penem in solution in a suitable inert solvent such as methylene chloride can be added to the DAC and TPP reagents under cooling e.g. −30°C to −10°C preferably −20°C. A fluoro or cyano ion source can then be added, for example sodium or potassium cyanide, or diphenoxycyanophosphine, or a solution of hydrogen fluoride in pyridine.

Alternatively, for example, a suitably protected penem can be reacted with a dialkylaminosulfur trifluoride preferably diethylaminosulfur trifluoride, usually in the presence of an inorganic base, e.g. calcium carbonate, to convert the hydroxy to fluoro.

Generally, in the process of the invention, the carboxy group of the compounds [IX(a), IX(b)], XI and XIII, and the starting penem of process D will be protected in the respective process. Thus, for instance, the alkylation reaction of process A will give a carboxy protected derivative of the respective compound of formula I which has subsequently to be deprotected. The hydroxy group of the hydroxyalkyl attached to the 3-position of the azetidinone ring of the said compounds may also, conveniently, be protected.

Suitable hydroxy protecting groups for use in the process of this invention are such groups conventionally used for such purpose in the β-lactam art and which are readily removable by procedures utilizing elemental zinc or any other conventional procedures. Preferred hydroxy protecting groups are trichloroethoxycarbonyl, dimethyltributylsilyl, trimethylsilyloxycarbonyl and trimethylsilyl:trimethylsilyl is a particularly preferred protecting group and is removable by treatment with a mild aqueous acid, such as aqueous acetic acid.

Suitable carboxy protecting groups are those conventionally used in the penem art and which can be removed under conventional conditions without reaction with other functional groups present on the penem molecule, for example allylic, p-nitrobenzyl and trichloroethyl. The preferred carboxy protecting group is allyl.

The removal of the protecting group from a protected carboxyl group can be carried out by conventional procedures selected according to the identity of the protecting group. For the removal of the preferred protecting group, allyl, this can in general be effected under catalytic conditions in the presence of a base, preferably by utilizing procedures described in our European Patent Application Publication No. 0013663. Thus an allyl group is preferably removed by utilizing a suitable aprotic solvent, such as tetrahydrofuran, diethyl ether or methylene chloride, with an alkali metal alkylcarboxylate, preferably potassium or sodium 2-ethylhexanoate (to give the alkali metal penem salt, preferably the sodium or potassium penem salt directly) or carboxylic acid, preferably 2-ethylhexanoic acid (to give the penem free-acid) and a mixture of a palladium compound and triphenyl phosphine as a catalyst.

If the product is a zwitterion deprotection of the allylic group requires only the catalyst and any mild neucleophile (e.g. $H_2O$ or alcohol).

Preparation of the foregoing salts and esters may be carried out according to conventional procedures for forming salts of beta-lactams such as penicillins, cephalosporins and penems. Salts can be formed upon deprotection of an allyl group as above, or for example, by treating the free acid with metal compounds such as alkali metal salts of suitable carboxylic acids, or with ammonia or a suitable organic amine, wherein preferably stoichiometric amounts or only a small-excess of the salt-forming agent is used. Acid addition salts of the compound can be obtained in the usual manner, for example by treating a compound of formula I with an acid or a suitable anion exchange reagent. Zwitterions may be formed by neutralizing salts such as acid addition salts to the isoelectric point. The esters are preparable in a manner analogous to the preparation of the corresponding esters of penicillins and cephalosporins.

Salts may be converted in the usual manner into the free carboxy compounds.

The compounds may be prepared as their racemic mixtures, *e.g.,* a 5R,6S,8R compound is produced with its enantiomer (mirror image), *i.e.,* a 5S,6R,8S compound, in equal amounts when the starting compound is a racemic mixture. The two enantiomers may, if desired, be separated by conventional means, *e.g.,* by fractional crystallizations of optically active salt forms, *e.g.,* the salts derived from optically active amino compounds, *e.g.,* (−)-brucine, or (+)- and (−)-ephedrine.

Alternatively, the compounds may be produced in optically active form by utilizing optically active starting material in the reaction procedures.

The starting materials for the procedures described above are either known in the art or can be prepared by standard methods.

The compounds of formula X in which $R^1$ is loweralkyl substituted by groups such as cyano or halogen may be prepared from compounds of the formula XIII

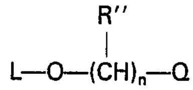

XIII

in which R'' is hydroxyloweralkyl, n and Q are as defined above and L is a hydroxy protecting group. The hydroxy of the R'' group is mesylated e.g. by reaction with mesylchloride in dry pyridine at room temperature, and the mesyl group then displaced by reaction with an appropriate cyano or halogen containing reagent e.g. sodium cyanide or hydrogen fluoride. The protected oxygen can then be deprotected and displaced by or functionalized to the appropriate Z group e.g. it may be mesylated by reaction with mesylchloride as mentioned above for the R'' group, or converted to halide by reaction with a hydrogen halide.

The tautomeric compound of formula [IX(a),IX(b)] can be prepared by the methods disclosed in European Patent Application No. 83111615.7.

A description of a suitable procedure is given below in which for convenience compounds of 5R,6S,8R stereochemistry and a 6-(1-hydroxyethyl substituent is produced. It will be apparent to the skilled man, however, that compounds of other stereoconfigurations and other hydroxyalkylsubstituents at the 6-position, as well as with carboxy protecting groups other than allyl, can be prepared using the same method.

This procedure, in a preferred form, comprises:

a) reacting an azetidinome of formula XX

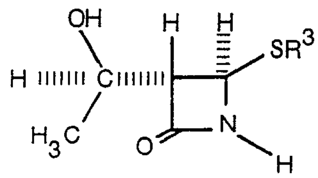

XX

in which $R^3$ is a sulfur protecting group, e.g. triphenylmethyl, with an α-substituted alkylacetate of formula XXI

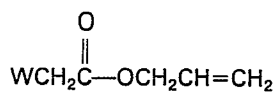

XXI

in which W is a leaving group e.g. iodo or bromo to form a compound of formula XXII

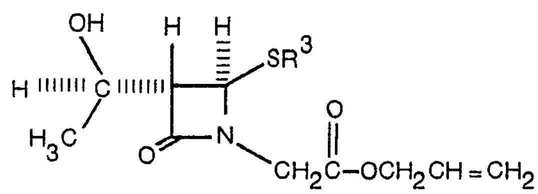

XXII

This reaction can be carried out at 15° to 30°C in the presence of an acid acceptor. Preferably the reaction is conducted in acetonitrile employing cesium carbonate or tetraalkyl ammonium hydroxide as the acid acceptor;

b) treating the compound of formula XXII with a stoichliometric excess of elemental zinc in hydrochloric acid in an suitable organic solvent such as tetrahydrofuran at 15°C to 25°C, to form the compound of formula XXIII

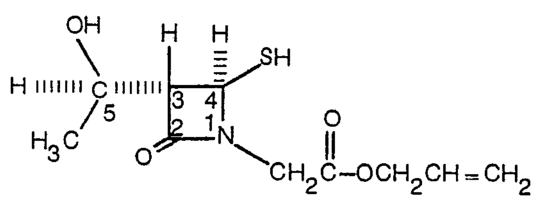

XXIII

c) protecting the hydroxy group at the 5 position with a trimethylsilyl group by reaction of compound of formula XXIII with bis-trimethyl silylacetamide; the reaction suitably being carried out in a solvent such a dimethylformamide at 0°C to 30°C;

d) reacting the resulting protected compound with a thiocarbonyl compound of formula XXIV

$$S=C(Y)_2$$ XXIV

in which Y is a leaving group such as halogen naphthyloxy or imidazolyl, generally using the same solvent as in the preceding step at a temperature in the range of 10°C to 45°C to form the compound XXV

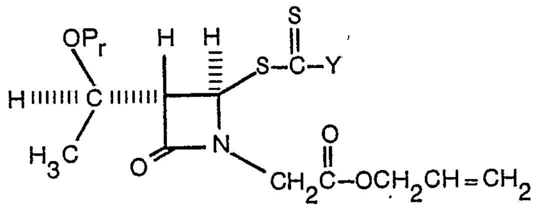

XXV

in which $P_r$ is trimethylsilyl and Y is as defined above;

e) treating compound XXV with an equimolar amount of a strong base such as lithium diisopropyl amide, to produce a compound [IX(a),IX(b)]; the reaction will generally be carried out in an anhydrous inert organic solvent, such as tetrahydrofuran, with the base being added to a solution of compound XXV in the solvent; typical reaction temperatures are −50°C to −100°C, and the reaction will generally be complete within 5 minutes to 24 hours.

The compounds of formula XIII can be obtained by following the procedure described in European Patent Application publication No. 58317, e.g. by reacting of a compound of the formula

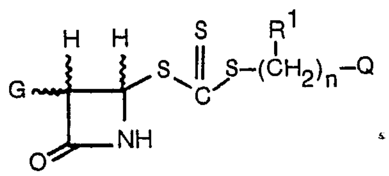

in which G, *n* and Q are as defined above, with a reactive derivative, e.g. chloride, of an acid of the formula

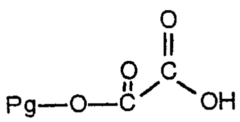

in which Pg is a carboxy protecting group as defined above. This reaction is usually carried out under normal acylating conditions, namely in an inert solvent and in the presence of an organic base, preferably a tertiary amine.

Typical examples of compounds of the present invention are:

5R,6S,8R-2-[2-(imidazol-1-yl)ethylthio]-6-(1-hydroxyethyl)-penem-3-carboxylic acid,
5R,6S,8R-2-[2-(1,2,4-1*H*-triazol-1-yl)ethylthio]-6-(1-hydroxyethyl)-penem-3-carboxylic acid, sodium salt,
5R,6S,8R-2-[2-(1,2,3-triazol-1-yl)ethylthio]-6-(1-hydroxyethyl)penem-3-carboxylic acid,
5R,6S,8R-2-[2-(2-methyl-5-nitroimidazol-1-yl)ethylthio]-6-(1-hydroxyethyl)penem-3-carboxylic acid,
5R,6S,8R-2-[2-(tetrazol-1-yl)ethylthio]-6-(1-hydroxyethyl)penem-3-carboxylic acid,
5R,6S,8R-2-[2-(tetrazol-2-yl)ethylthio]-6-(1-hydroxyethyl)penem-3-carboxylic acid,
5R,6S,8R-2-[1-(R,S)-methyl-2-(imidazol-1-yl)ethylthio]-6-(1-hydroxyethyl)penem-3-carboxylic acid.

## Preparation 1

Bromoethylimidazole

A) Dissolve 10 g imidazole and 20 g ethylene carbonate in 20 ml toluene, heat at reflux for 5 hours, cool, and remove the top layer. With cooling, add 15 ml concentrated hydrochloric acid to the bottom layer, then extract the excess ethylene carbonate with chloroform. Add potassium carbonate to make basic, extract with 5 × 50 ml chloroform, dry over sodium sulfate and evaporate the solvent *in vacuo.* Distill under reduced pressure to obtain hydroxyethylimidazole, b.p. 134—136°C/0.5 mm.

B) Dissolve 1.1 g of hydroxyethylimidazole in 10 ml chloroform. Cool in an ice bath, add a solution of 2.28 g thionyl bromide in 5 ml chloroform and stir for 30 minutes. Stir another hour at room temperature, then pour the resultant mixture into 15 ml cold water and neutralize with sodium bicarbonate. Separate the chloroform layer and retain; extract the aqueous layer with 15 ml chloroform. Combine the chloroform solution, dry over sodium sulfate and evaporate the solvent to obtain bromoethylimidazole.

## Preparation 1A

Bromoethylimidazole Hydrobromide salt

Dissolve 5.2 g of hydroxethylimidazole (preparation I, step A) in 25 ml methylene chloride. Cool in an ice bath, add 3.85 ml thionyl bromide in 10 ml methylene chloride and stir for 1 hour. Evaporate the solvent *in vacuo* and use the resultant residue without further purification.

## Preparation 2

Bromoethyltriazole Hydrobromide Salt

A) Dissolve 3.2 g potassium tert-butoxide in 40 ml methanol, add 2.0 g 1,2,4-triazole and 3.6 g 2-bromoethanol and stir overnight. Evaporate the solvent *in vacuo.* Dissolve the residue in ethyl acetate and purify by column chromatography (silica gel, elute with methylene chloride changing to 10% methanol in

methylene chloride). Combine fractions containing more polar product as shown by TLC and evaporate the solent *in vacuo* to obtain hydroxyethyltriazole.

B) Stir 600 mg of the product of step A and 1.1 g thionyl bromide in 6 ml of chloroform under a nitrogen atmosphere for 1½ hours. Evporate the solvent *in vacuo*. Dry the resultant residue under high vacuum for 15 minutes to obtain the title compound.

Preparation 3

(2-Hydroxy)propylimidazole

Mix 6.8 g imidazole and 15.3 g propylene carbonate and heat at 140°C for 1 hour. Chromatograph the resultant residue on silica gel, eluting with methylene chloride : ethyl acetate (1:1), then with 5→10% methanol in methylene chloride to obtain the title compound.

Preparation 4

3-Hydroxy-4-(imidazol-1-yl)butyronitrile

A) Cool to 0°C a solution of 9 g sodium imidazole in 40 ml dimethylformamide, add 13.7 g epibromohydrin in 40 ml dimethylformamide, and warm to room temperature for 30 minutes. Evaporate the solvent *in vacuo* and purify the resultant residue on silica gel, eluting with tetrahydrofuran, to obtain epiimidazole hydrin.

B) Dissolve 12.4 g of the product of step A in 25 ml dimethylsulfoxide, add 4.9 g sodium cyanide in 25 ml dimethylsulfoxide, and warm to 50°C. Evaporate the solvent *in vacuo* and purify the resultant residue on silica gel using tetrahydrofuran→20% methanol in tetrahydrofuran to obtain the title compound.

Preparation 5

1-Ethoxy-1-(2-mercaptohydroxy-3-(imidazol-1-yl)propoxy)ethane

A) Dissolve 40 g glycidol in 1 liter methylene chloride, add 40 g ethyl-vinyl-ether and cool to 2°C. Add 250 mg p-toluenesulfonic acid and warm slowly to room temperature. Evaporate the solvent *in vacuo* and distill the residue at 2 mm Hg (b.p. 39—41°C) to obtain 1-(2,3-epoxypropyloxy)-1-ethoxy)-ethane.

B) Dissolve 16.6 g imidazole in 75 ml dimethylformamide, add 35.82 g of the product of step A and heat slowly to 120°C. Cool the resultant mixture to room temperature. Evaporate the solvent *in vacuo*, partition the resultant residue between methylene chloride and water (125 ml/125 ml), extract once with water, then evaporate the organic layer *in vacuo* to obtain 1-ethoxy-1-(2-hydroxy-3-(imidazol-1-yl)-propoxy)ethane.

C) To 4.0 g of the product of step B in 30 ml methylene chloride add 3.2 ml triethylamide and 1.6 ml methanesulfonyl chloride. Stir at room temperature for 1 hour, then extract the resultant mixture with water (2 × 50 ml) and evaporate the organic layer *in vacuo* to obtain 1-ethoxy-1-(2-methyl-sulfonyloxy-3-(imidazol-1-yl)-propoxy)ethane.

D) To 4.25 g of the product of step C in 25 ml dimethylformamide, add 5.25 g sodium thioacetate, and stir 48 hours at room temperature. Evaporate the solvent *in vacuo*, dissolve the resultant residue in 100 ml methylene chloride, filter and evaporate the methylene chloride *in vacuo*. Dissolve the residue in 100 ml ethanol, saturate with ammonia and let stand at room temperature for 1 hour. Evaporate the solvent *in vacuo*, partition the resultant residue between water and methylene chloride, then remove the methylene chloride *in vacuo*. Purify the resultant reside on silica gel, eluting with ethyl acetate : methylene chloride (1:1) to obtain the title compound.

Preparation 6

A) Heat together for 2 hours, at 120°C, 6.8 g imidazole and 16 g ethylene carbonate. Cool the reaction mixture and purify by chromatography using a silica gel column (100% methylene chloride→100% ethylacetate) to give N-hydroxyethylimidazole.

B) Dissolve 10 g of N-hydroxyethylimidazole in 100 ml methylene chloride. Cool the solution to 0°C and add 1.2 equivalents of triethylamine, followed by 1.1 equivalents of methylsulfonylchloride added over 5 minutes. Warm the reaction mixture to room temperature and stir for 1 hour. Partition the resulting reaction mixture between water and methylene chloride. Collect the organic layer and remove the methylene chloride to give N-methylsulfonyloxyethylimidazole.

C) Dissolve 10 g of N-methylsulfonyloxyethylimidazole in 25 ml dimethylformamide. Add 2 equivalents of sodium thio-acetate and stir at room temperature for 48 hours. Remove the solvent *in vacuo*, add methylene chloride, filter and remove the methylene chloride *in vacuo*. Dissolve the residue in 200 ml ethanol and saturate with ammonia. Allow the solution to stand at room temperature for 1 hour. Remove the ethanol *in vacuo*. Purify on silica gel using 100% methylene chloride→100% ethylacetate to give N-mercaptoethylimidazole.

D) To a solution of 10 g N-mercaptoethylimidazole in 50 ml $H_2O$ and 50 ml ethanol add 1 equivalent sodium hydroxide and 10 ml of CS2. Stir the reaction mixture for one hour to obtain a solution containing the imidazole derivative of the formula A

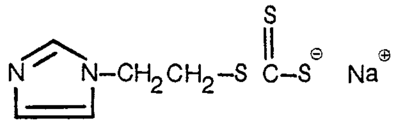

A

E) Charge to a 250 ml flask 5 g (0.0143M) 3-(1-(2,2,2-trichloroethyloxycarbonyloxy)ethyl)-4 acetoxy-azetidin-2-one and 50 ml methylene chloride. Add 1.5 equivalents of the imidazole derivative (formula A) from step D in aqueous ethanol to the mixture in the flask and stir the resulting mixture for 1 hour. Chromatograph the reaction mixture on silica gel to isolate 3-(1-(2,2,2-trichloroethyloxycarbonyloxy)ethyl)-4(2-imidazol-1-yl)ethylthiocarbothioylthio)-azetidin-2-one.

F) Charge the azetidinone isolate from step E together with 50 ml methylene chloride to a 100 ml flask. Cool the mixture to −20°C and add 1.2 equivalents of allyloxalylchloride to the mixture. Add 1.2 equivalents diisopropyl ethylamine (Münings base) in methylene chloride over 5 minutes and stir for 45 minutes. Wash the reaction mixture with $H_2O$ to obtain a compound having the formula B

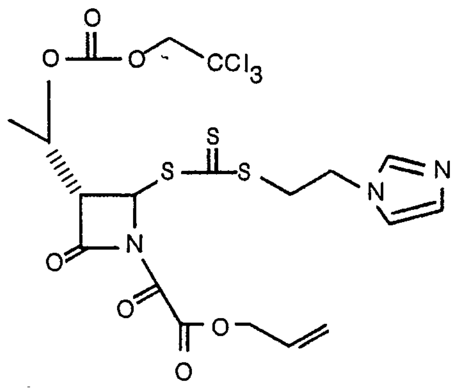

B

Example 1

(5R,6S,8R)-2-[2-(Imidazol-1-yl)ethylthio]-6-(1-hydroxyethyl)penem-3-carboxylic acid

A) To a solution of 0.5 g of allyl (5R,6S,8R)-2-thiol-6-(1-hydroxyethyl)penem-3-carboxylate and allyl (5R,6S,8R)-2-thione-6-(1-hydroxyethyl)penem-3-carboxylate in 10 ml tetrahydrofuran (THF), add 0.7 g bromoethylimidazole, followed by 2.0 ml of a 5% aqueous solution of sodium bicarbonate, and stir at room temperature for 3 hours or until thin layer chromatography (ethyl acetate/THF, 50/50) indicates no starting material is left. Evaporate the THF *in vacuo*, add 10 ml water to the resultant residue and extract with 2—20 ml methylene chloride. Dry the organic layer over sodium sulfate and concentrate *in vacuo* to an oil. Purify the crude oil by column chromatography (silica gel, eluted with 5% methanol in methylene chloride) to obtain 100 mg of allyl (5R,6S,8R)-2-[2-(imidazol-1-yl)-ethylthio]-6-(1-hydroxyethyl)penem-3-carboxylate.

B) Under argon, add 100 mg of the allyl ester product of step A) to 10 ml methylene chloride, followed by 25 mg triphenyl phosphine, 45 mg 2-ethyl hexanoic acid, and 10 mg Pd° reagent. Allow the mixture to stand until the reaction is complete as shown by thin layer chromatography (5% methanol in methylene chloride as solvent). Extract the resultant product with 2 × 10 ml water, then wash the aqueous layer with 3 × 10 ml methylene chloride. Lyophilize the aqueous layer to obtain 73 mg of crude title compound.

Purify the product by reverse phase column chromatography (25 g silica gel eluted with water) to obtain 53 mg of the title compound.

NMR—($D_2O$) = 8.65 (1H, s), 7.4 (2H, d), 5.3 (1H, s), 4.4 (2H, m), 4.05 (1H, m), 3.7 (1H, d), 3.3 (2H, m), 1.5 (3H, d).

Example 1A

(5R,6S,8R)-2-[2-(Imidazol-1-yl)ethylthio]-6-(1-hydroxyethyl)penem-3-carboxylic acid

A) Dissolve 3.0 g of allyl(5R,6S,8R)-2-thiol-6-(hydroxyethyl)penem-3-carboxylate and allyl(5R,6S,8R)-2-thione-6-(1-hydroxyethyl)penem-3-carboxylate in 30 ml THF and 10 ml water. Add a solution of 5.12 g bromoethylimidazole hydrobromide salt (preparation 1A) in 20 ml THF and 20 ml water, followed immediately by 3 g sodium bicarbonate in 10 ml water. Stir at room temperature for 2½ hours or until thin layer chromatography (30% ethyl acetate in methylene chloride) indicates the reaction is complete. Add 100 ml water to the resultant mixture and extract with 2 × 100 ml ethyl acetate. Dry the organic layer over sodium sulfate and concentrate *in vacuo*.

Purify as in Example 1 to obtain allyl((5R,6S,8R)-2-[2-(imidazol-1-yl)-ethylthio]-6-(1-hydroxyethyl)-penem-3-carboxylate.

B) Carry out the procedure described in Example 1, step B to obtain the title compound.

Example 2

(5R,6S,8R)-2-[2-(1,2,4-1H-Triazol-1-yl)-ethylthio]-6-(1-hydroxyethyl)penem-3-carboxylic acid

A) Proceed similarly to the procedure of Example 1A, step A, first paragraph, but substitute bromoethyltriazole hydrobromide salt (preparation 2) for the bromoethylimidazole HBr salt. Then slurry in hot ethyl acetate, cool and filter to obtain allyl(5R,6S,8R)-2-[(1,2,4-1H-triazol-1-yl)ethylthio]-6-(1-hydroxyethyl)-penem-3-carboxylate.

NMR—90 mHz ($CD_3CN$)— = 8.22 (1H, s), 7.92 (1H, s), 6.3—5.6 (1H, m), 5.69 (1H, d, J=1.5Hz), 5.65—5.08 (2H, m), 4.8—4.6 (2H, m), 4.5—4.4 (2H, m), 4.3—3.9 (1H, m), 3.78 (1H, dd, J=1.5Hz, 6Hz), 3.6—3.2 (2H, m), 1.23 (3H, d, J=6Hz)

B) Under nitrogen, stir 160 mg of the product of step A, 0.1 ml pyridine and 0.265 ml 2-ethylhexanoic acid in 6 ml acetonitrile for 1 hour. Evaporate the solvent *in vacuo* at 40°C or lower. Add methylene chloride and water to the resultant residue. Separate the water layer and extract the methylene chloride layer with 3 × 20 ml water. Combine the aqueous extracts and wash with methylene chloride. Evaporate the water *in vacuo* to obtain the title compound.

NMR—90 mHz ($D_2O$)— = 8.5 (1H, s), 8.13 (1H, s), 5.51 (1H, d, J=1.5Hz), 4.7—4.5 (2H, m), 4.4—4.05 (1H, m), 3.83 (1H, dd, J=1.5, 6Hz), 3.7—3.15 (2H, m), 1.29 (3H, d, J=6Hz).

Example 3

(5R,6S,8R,2(R,S))-2-[1-Methyl-2-(imidazol-1-yl)ethylthio]-6-(1-hydroxyethyl)penem-3-carboxylic acid

A) Dissolve 0.5 g of 2-(hydroxy)propylimidazole (Preparation 3) in 2 ml methylene chloride and add the resultant solution to a mixture of 0.8 ml trifluoromethanesulfonic anhydride and 0.83 ml diisopropylethylamine in 10 ml methylene chloride at 0°C. Stir for 30 minutes, then add the resultant mixture to a solution of 1 g of allyl(5R,6S,8R)-2-thiol-6-(1-hydroxyethyl)penem-3-carboxylic acid and allyl(5R,6S,8R)-2-thione-6-(1-hydroxyethyl)penem-3-carboxylate in 10 ml tetrahydrofuran, 10 ml water, and 0.3 g sodium bicarbonate. Stir at room temperature until thin layer chromatography (methanol : methylene chloride, 1:9) indicates no starting material is left. Evaporate the solvent *in vacuo* and partition the resultant residue between water and methylene chloride. Dry the organic layer over magnesium sulfate and evaporate the solvent *in vacuo*. Purify the resultant residue using silica gel, eluting with 25% ethylacetate/methylene chloride→100% ethyl acetate to obtain allyl(5R,6S,8R,2(R,S))-2-[1-methyl-2-(imidazol-1-yl)ethylthio]-6-(1-hydroxyethyl)penem-3-carboxylate.

B) Treat the product of step A in a manner similar to that described in Example 2, step B to obtain the title compound.

Example 4

(5R,6S,8R,2(R,S))-2-[1-Cyanomethyl-2-(imidazo-1-yl)ethylthio]-6-(1-hydroxyethyl)penem-3-carboxylic acid

A) Treat the product of Preparation 4 in a manner similar to that described in Example 3, step A, first sentence, but substituting triethylamine for diisopropylethylamine and cooling to −78°C. Then proceed as further described in Example 3, step A, carrying out the reaction with the thiol and thione at 0°C to obtain allyl(5R,6S,8R,2(R,S))-2-[1-cyanomethyl-2-(imidazol-1-yl)ethylthio)-6-(1-hydroxyethyl)penem-3-carboxylate.

Alternatively, dissolve the residue of preparation 4, step B in 50 ml methylene chloride at 0°C, add 1.2 equivalents of triethylamine, 1 equivalent of methanesulfonyl chloride, and warm to room temperature for 30 minutes. Evaporate the solvent *in vacuo*, extract the resultant residue with tetrahydrofuran and continue with the reaction with the thiol and thione as described in Example 4, step A, above.

B) Treat the product of step A in a manner similar to that described in Example 2, step B to obtain the title compound.

Example 5

(5R,6S,8R,2(R,S))-2-[1-Hydroxymethyl-2-(imidazol-1-yl)ethylthio]-6-(1-hydroxyethyl)penem-3-carboxylic acid

A) Dissolve 2 g of allyl (5R,6S,8R)-2-ethylthio-6-(1-hydroxyethyl)penem-3-carboxylate in 80 ml methylene chloride at −5°C, add 2 g calcium carbonate, then 1.1 g m-perbenzoic acid in 20 ml methylene chloride. After 20 minutes extract the reaction mixture with 100 ml water, separate and evaporate the organic layer to obtain allyl(5R,6S,8R)-2-ethylsulfinyl-6-(1-hydroxyethyl)penem-3-carboxylate.

B) Dissolve 500 mg of the product of step A in a mixture of 4 ml acetonitrile and 2 ml water, add 100 mg sodium bicarbonate, then 700 mg of the product of preparation 5 and let stand 1 hour. Partition the resultant mixture between methylene chloride and water, evaporate the methylene chloride and purify the resultant residue on a silica gel column using methylene chloride : tetrahydrofuran (1:1) to obtain allyl(5R,6S,8R,2(R,S))-2-[1-(ethoxy-1-ethoxy)-methyl-2-(imidazol-1-yl)ethylthio]-6-(hydroxyethyl)penem-3-carboxylate.

Alternatively, react the mesylate product of preparation 5, step C, allyl(5R,6S,8R)-2-thiol-6-(1-hydroxyethyl)penem-3-carboxylate and allyl(5R,6S,8R)-2-thione-6-(1-hydroxyethyl)penem-3-carboxylate, in a manner similar to the reaction described in Example 3, step B.

C) Dissolve 174 mg of the product of step B in 10 ml of a mixture of water : terahydrofuran (1:1), then adjust to pH 3 with a 5% aqueous solution of p-toluenesulfonic acid. Let the mixture stand for 1.5 hours, evaporate the solvent *in vacuo* and partition the resultant residue between 5% aqueous sodium bicarbonate and methylene chloride. Evaporate the organic layer, redissolve the resultant residue in 10 ml methylene chloride and continue using the procedure described in Example 1, step B, to obtain the title compound.

Example 6

(5R,6S,8R,2(R,S))-2-[1-Fluoromethyl-2-(imidazol-1-yl)ethylthio]-6-(1-hydroxyethyl)penem-3-carboxylic acid

A) Dissolve 0.100 g of the product of Example 5, step C obtained before carrying out the procedure of Example 1, step B, said product being allyl(5R,6S,8R,2(R,S))-2-[1-hydroxymethyl-2-(imidazol-1-yl)-ethylthio]-6-(1-hydroxyethyl)penem-3-carboxylate, together with 0.05 g of calcium carbonate in 5 ml of

methylene chloride at −78°C. Add 0.043 g of diethylaminosulfur trifluoride and stir for 30 minutes. Dilute with ethyl acetate, then stir with water for 5 minutes at 0°C. Separate the organic layer, wash with water and evaporate the solvent. Chromatograph the resultant residue on silica gel, eluting with methylene chloride : ethyl acetate to obtain allyl(5R,6S,8R,2(R,S))-2-[1-fluoro-methyl-2-(imidazol-1-yl)ethylthio]-6-(1-hydroxyethyl)penem-3-carboxylate.

B) Treat the product of step A in a manner similar to that described in Example 1, step B, to obtain the title compound.

Example 7

(5R,6S,8R,2(R,S))-2-[2-Hydroxy-3-(imidazol-1-yl)propylthio]-6-(1-hydroxyethyl)penem-3-carboxylic acid

A) Slowly combine epiimidazolohydrin as prepared in Preparation 4, step A with 600 mg of allyl(5R,6S,8R,)-2-thiol-6-(1-hydroxyethyl)penem-3-carboxylate and allyl(5R,6S,8R,)-2-thione-6-(1-hydroxyethyl)penem-3-carboxylate in 2 ml dimethylformamide and stir until thin layer chromatography (on silica gel elute with methanol : methylene chloride (1:9)) shows no more thione present. Partition the resultant solution between water and methylene chloride, evaporate the organic layer and purify the resultant residue on a silica gel column, eluting with 5→10% ethanol in methylene chloride.

B) Treat the product of step A in a manner similar to that described in Example 1, step B, to obtain the title compound.

Example 8

(5R,6S,8R-2-[2-(Imidazol-1-yl)ethylthio]-6-(1-hydroxyethyl)penem-3-carboxylic acid

A) Charge 5 g of compound of formula B (preparation 6) together with 50 ml distilled methylene chloride. Heat to reflux and add over 2 hours two equivalents of triethyl phosphite. Reflux for 4 hours by which time cyclisation is complete. Isolate the cyclised product by chromatography on silica gel to obtain a compound having the formula C

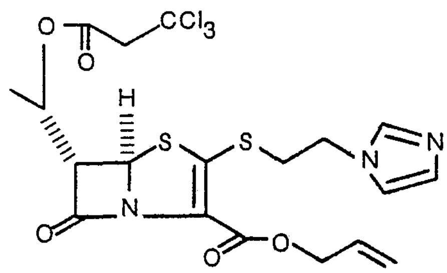

C

B) Dissolve 1.6 g of the compound of formula C in a mixture consisting of 1.5 ml acetic acid, 1.5 ml water and 15 ml tetrahydrofuran. Add 1.25 g zinc dust and stir the mixture at 0° to 5°C. After 1 hour filter, wash the filtrate with saturated sodium bicarbonate solution. Dry the filtrate over magnesium sulfate, filter and evporate to a residue. Crystallize the residue from ethylether : methylene chloride to obtain compound of formula D

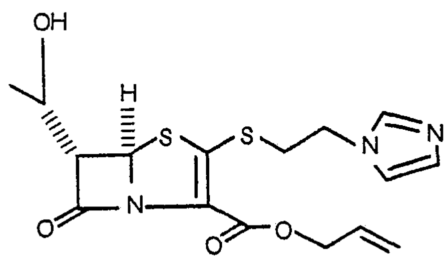

D

C) Carry out the procedure of Example 1, step B, to obtain the title compound.

By following the procedures outlined in the above examples, and conventional procedures for salt and ester formation, the following compounds of this invention may be prepared:

Compounds of the formula

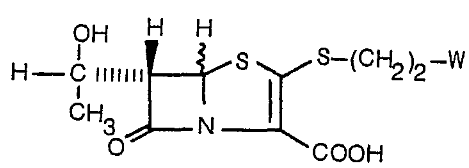

wherein W is

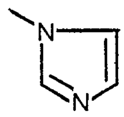 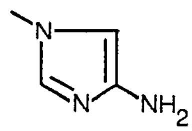 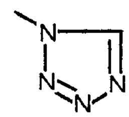 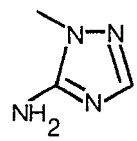

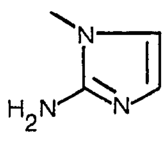 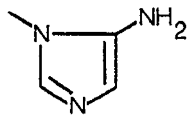 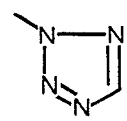 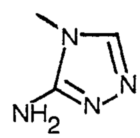

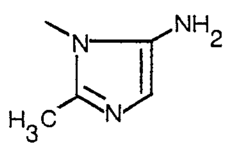 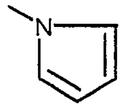 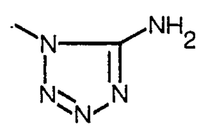 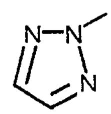

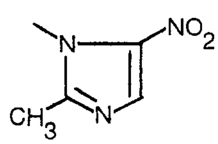 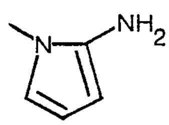 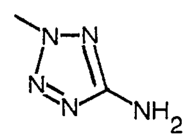 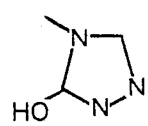

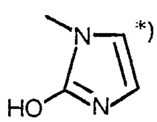 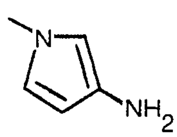 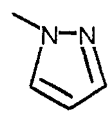 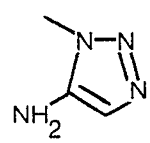

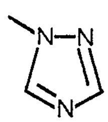 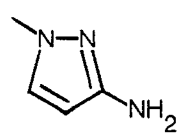 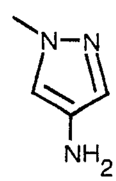 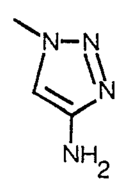

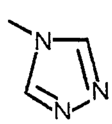 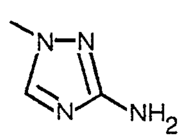 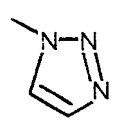 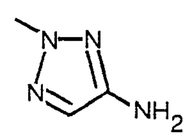

*) ketoform

and sodium and potassium salts or pharmaceutically acceptable esters thereof.

Compounds of the formula

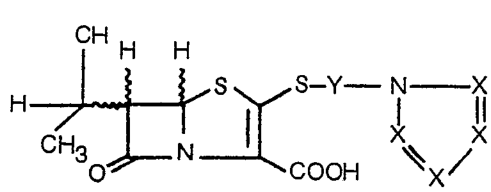

wherein X is as defined above and Y is one of the following radicals (drawn so as to read from left to right):

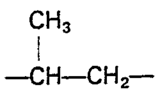 ,

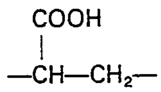 ,

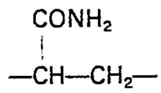 ,

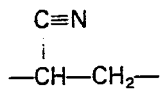 ,

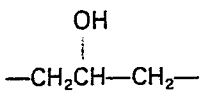 ,

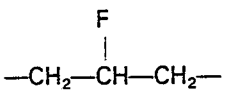 ,

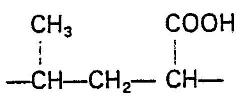 ,

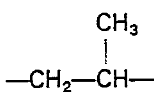 ,

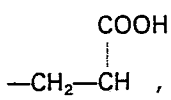 ,

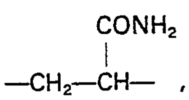 ,

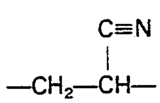 ,

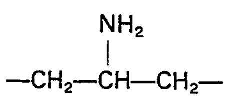 ,

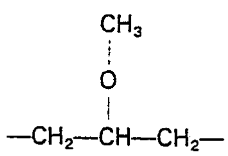 ,

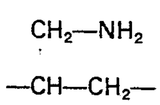

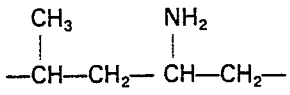 ,

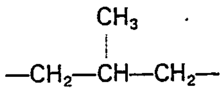 ,

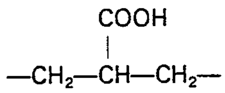 ,

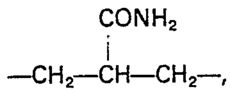 ,

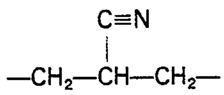 ,

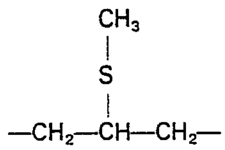 ,

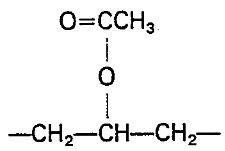 ,

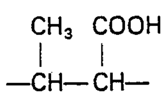 ,

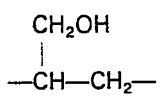

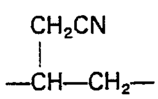

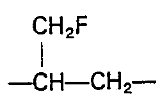

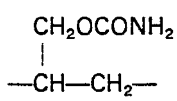

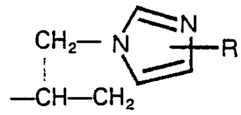

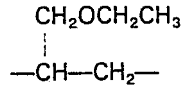

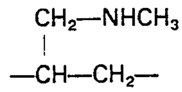

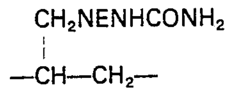

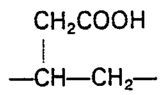

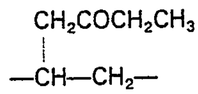

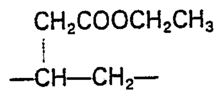

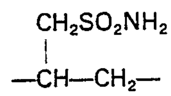

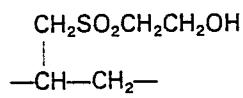

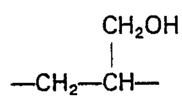

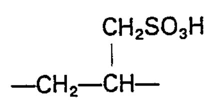

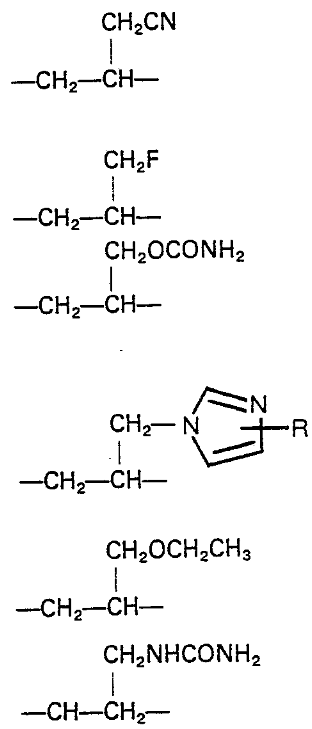

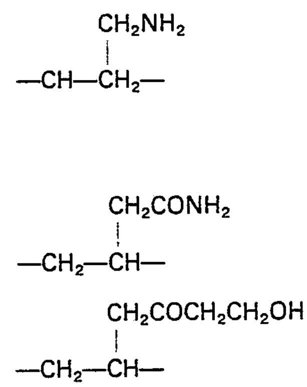

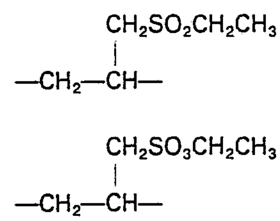

and sodium and potassium salts and pharmaceutically acceptable esters thereof.

In the following examples the Active Ingredient is 5R,6S,8R-2-[2-(imidazol-1-yl)ethylthio]-6-(1-hydroxyethyl)penem-3-carboxylic acid or an equivalent amount of any of its pharmaceutically acceptable salts and esters, or any of the other compounds of formula 1, mentioned above, or their salts or esters.

Example 9

Injectable Solution

| Ingredient | mg/ml | mg/ml |
|---|---|---|
| Active Ingredient | 100 | 500 |
| Methylparaben | 1.8 | 1.8 |
| Propylparaben | 0.2 | 0.2 |
| Sodium Bisulfite | 3.2 | 3.2 |
| Disodium Edetate | 0.1 | 0.1 |
| Sodium Sulfate | 2.6 | 2.6 |
| Water for Injection q.s. ad | 1.0 ml | 1.0 ml |

Method of Manufacture

1. Dissolve the parabens in a portion (85% of the final volume) of the water for injection at 65—70°C.
2. Cool to 25 to 35°C. Charge and dissolve the sodium bisulfite, disodium edetate and sodium sulfate.
3. Charge and dissolve the active ingredient.
4. Bring the solution to final volume by adding water for injection.
5. Filter the solution through 0.22 μ membrane and fill into appropriate containers.
6. Terminally sterilize the units by autoclaving.

Example 10

Injectable Powder: (per vial)

| | g/vial | g/vial |
|---|---|---|
| Active Ingredient | 0.5 | 1.0 |

Add sterile water for injection or bacteriostatic water for injection for reconstitution.

**Claims for the Contracting States: BE CH DE FR GB IT LI LU NL SE**

1. A compound of the formula

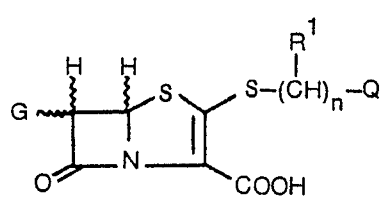

I

wherein

G is hydroxyloweralkyl,

Q is the group

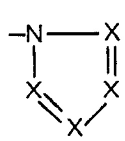

wherein each X is

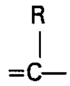

or =N—, with the proviso that at least one X is

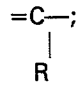

R is hydrogen, loweralkyl, aminoloweralkyl, mono- and di-loweralkylaminoloweralkyl, carboxyloweralkyl, sulfoloweralkyl, hydroxyloweralkyl, cyano, hydroxy, amino, mono- and di-loweralkylamino, loweralkylsulfonate, sulfamyl, halogeno, hydroxyliminoloweralkyl, loweralkoxyiminoloweralkyl, carboxy, carbamyl, mono- or di-loweralkylcarbamyl, nitro, carbamyloxy, ureidoloweralkyl, or carbamylhydrazoloweralkyl; or two R groups attached to adjacent carbon atoms, together with the carbon atoms to which they are attached, form a fused aromatic ring;

each $R^1$ is independently hydrogen, loweralkyl, carboxy, carbamyl, cyano, hydroxy, amino, loweralkylthio, fluoro, loweralkoxy, loweralkanoyloxy, or loweralkyl substituted by imidazolyl (which may be substituted by a group defined by R or by ureido), amino, loweralkylamino, carbamoylamino, ureidoamino, hydroxy, cyano, halogen, loweralkoxy, carbamyloxy, carboxy, carbamyl, loweralkylcarbonyl, sulfo, sulfamyl, loweralkylsulfonyl, loweralkoxysulfonyl, loweralkoxycarbonyl, hydroxyloweralkylcarbonyl

or hydroxyloweralkylsulfonyl, with the proviso that when $R_1$ is attached to a carbon atom adjacent either to the sulphur atom connected to the 2-position of the penem ring or the nitrogen atom by which the group Q is connected to the side chain

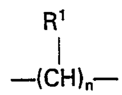

then such $R_1$ cannot be hydroxy, amino or fluoro;

n is 1 to 4;

and wherein the term "lower" denotes a radical having 1 to 6 carbon atoms; and

the pharmaceutically acceptable salts and esters thereof, in racemic or optically active form.

2. A compound as defined in Claim 1, characterized in that Q is chosen from pyrrolyl, imidazolyl, pyrazolyl, 1,2,4-triazolyl, 4,1,2-triazolyl, 1,2,3-triazolyl, 2,1,3-triazolyl, 1,2,3,4-tetrazolyl and 2,1,3,4-tetrazolyl, said group Q being unsubstituted or substituted by 1 to 4 R groups as defined in claim 1.

3. A compound as defined in claim 1 or 2, characterized in that R is chosen from hydrogen, amino, hydroxy and loweralkyl.

4. A compound as defined in any one of claims 1 to 3, characterized in that Q is a substituted or unsubstituted imidazolyl.

5. A compound as defined in claim 4, characterized in that Q is unsubstituted imidazolyl.

6. A compound as defined in any one of the preceding claims, characterized in that n is 2 and $R^1$ is hydrogen or a substituted lower alkyl group as defined in claim 1.

7. A compound as defined in Claim 1, namely:

5R,6S,8R-2-[2-(imidazol-1-yl)ethylthio]-6-(1-hydroxyethyl]penem-3-carboxylic acid,

5R,6S,8R-2-[2-(1,2,4-1*H*-triazol-1-yl)ethylthio]-6-(1-hydroxyethyl)-penem-3-carboxylic acid,

5R,6S,8R-2-[2-(1,2,3-triazolyl-1-yl)ethylthio]-6-(1-hydroxyethyl)penem-3-carboxylic acid,

5R,6S,8R-2-[2-(2-methyl-5-nitroimidazol-1-yl)ethylthio]-6-(1-hydroxyethyl)penem-3-carboxylic acid,

5R,6S,8R-2-[2-(tetrazol-1-yl)ethylthio]-6-(1-hydroxyethyl)penem-3-carboxylic acid,

5R,6S,8R-2-[2-(tetrazol-2-yl)ethylthio-6-(1-hydroxyethyl)penem-3-carboxylic acid,

5R,6S,8R-2-[1-(R,S)-methyl-2-(imidazol-1-yl)ethylthio]-6-(1-hydroxyethyl)penem-3-carboxylic acid,

or a pharmaceutically acceptable salt or ester of any one of the foregoing compounds.

8. A pharmaceutical composition comprising a compound as defined in any one of the preceding claims in admixture with a pharmaceutically acceptable carrier or excipient.

9. A composition as defined in claim 8, characterized in that the compound is 5R,6S,8R-2-[2-(imidazol-1-yl)-ethylthio)-6-(1-hydroxyethyl)penem-3-carboxylic acid or a pharmaceutically acceptable salt or ester thereof.

10. A process for the preparation of a compound as defined in claim 1, characterized in that

A) compound of formula [IX(a), IX(b)]

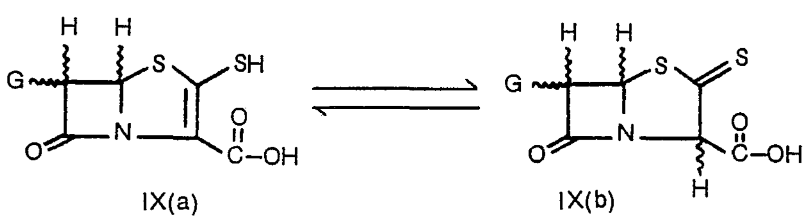

IX(a) IX(b)

in which G is as defined above, is transformed into a compound of formula I by introducing the group

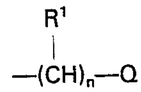

(in which Q, *n* and $R^1$ are as previously defined) by

(i) reacting a compound of formula X

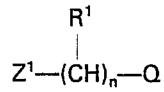

in which Q, $R^1$ and n are as defined above and $Z^1$ is a leaving group with the tautomer of formula IX(a) and IX(b), or (ii) reacting a compound of formula $X^1$

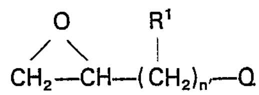

in which Q and $R^1$ are as defined above and n′ is zero to 2, with the tautomer of formulas IX(a) and IX(b)

B) Reaction of a compound of the general formula XI

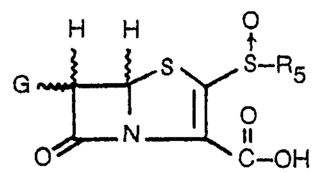

XI

in which G is as defined above and $R_5$ is an organic group, with a compound of general formula XII

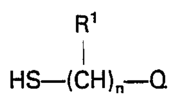

XII

in which Q, *n* and $R^1$ are as previously defined, or with a reactive derivative thereof, wherein the group $R_5$ is different from the group

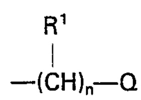

by which it is replaced,

C) intramolecular cyclisation of a compound having the general formula XIII

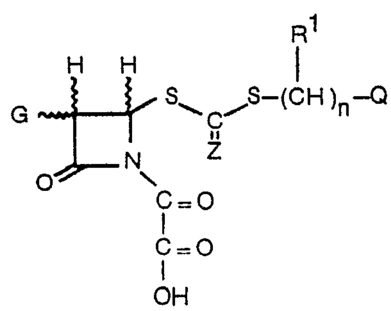

XIII

in which G, n, $R^1$ and Q are as defined above and Z is oxygen or sulphur, in the presence of a trivalent organophosphorous compound;

D) for the preparation of a compound of formula I in which at least one $R^1$ is cyano or fluoro, converting at least one hydroxyl group representing $R^1$ in a compound of formula I into the cyano or fluoro group;

wherein in the processes A, B, C or D, any functional groups are protected if necessary or desired,

the process A, B, C, or D, being followed by removal of any protecting groups, before or after any desired separation of a stereoisomer, and isolation of the resulting penem compound of formula I as the free acid, pharmaceutically acceptable salt or pharmaceutically acceptable ester.

**Claims for the Contracting State: AT**

1. A process for the preparation of a compound of the formula

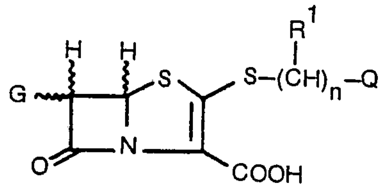

I

wherein

G is hydroxyloweralkyl,

Q is the group

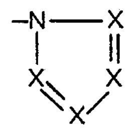

wherein each X is

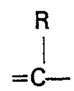

or =N—, with the proviso that at least one X is

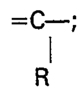

R is hydrogen, loweralkyl, aminoloweralkyl, mono- and di-loweralkylaminoloweralkyl, carboxyloweralkyl, sulfoloweralkyl, hydroxyloweralkyl, cyano, hydroxy, amino, mono- and di-loweralkylamino, loweralkylsulfonate, sulfamyl, halogeno, hydroxyliminoloweralkyl, loweralkoxyiminoloweralkyl, carboxy, carbamyl, mono- or di-loweralkylcarbamyl, nitro, carbamyloxy, ureidoloweralkyl, or carbamylhydrazoloweralkyl; or two R groups attached to adjacent carbon atoms, together with the carbon atoms to which they are attached, form a fused aromatic ring;

each $R^1$ is independently hydrogen, loweralkyl, carboxy, carbamyl, cyano, hydroxy, amino, loweralkylthio, fluoro, loweralkoxy, loweralkanoyloxy, or loweralkyl substituted by imidazolyl (which may be substituted by a group defined by R or by ureido), amino, loweralkylamino, carbamoylamino, ureidoamino, hydroxy, cyano, halogen, loweralkoxy, carbamyloxy, carboxy, carbamyl, loweralkylcarbonyl, sulfo, sulfamyl, loweralkylsulfonyl, loweralkoxysulfonyl, loweralkoxycarbonyl, hydroxyloweralkylcarbonyl

or hydroxyloweralkylsulfonyl, with the proviso that when $R_1$ is attached to a carbon atom adjacent either to the sulphur atom connected to the 2-position of the penem ring or the nitrogen atom by which the group Q is connected to the side chain

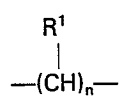

then such $R_1$ cannot be hydroxy, amino or fluoro;

n is 1 to 4;

and wherein the term "lower" denotes a radical having 1 to 6 carbon atoms; and

the pharmaceutically acceptable salts and esters thereof, in racemic or optically active form, characterized in that

A) compound of formula [IX(a), IX(b)]

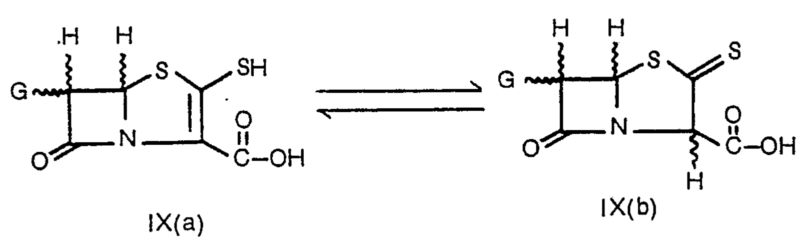

IX(a) IX(b)

in which G is as defined above, is transformed into a compound of formula I by introducing the group

$$-(\overset{\overset{\textstyle R^1}{|}}{CH})_n-Q$$

(in which Q, *n* and $R^1$ are as previously defined) by

(i) reacting a compound of formula X

$$Z^1-(\overset{\overset{\textstyle R^1}{|}}{CH})_n-Q \qquad \text{X}$$

in which Q, $R^1$ and n are as defined above and $Z^1$ is a leaving group with the tautomer of formula IX(a) and IX(b), or (ii) reacting a compound of formula $X^1$

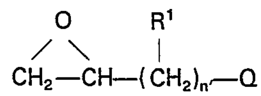

in which Q and $R^1$ are as defined above and n′ is zero to 2, with the tautomer of formulas IX(a) and IX(b)

B) Reaction of a compound of the general formula XI

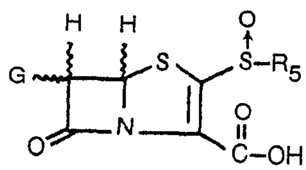

XI

in which G is as defined above and $R_5$ is an organic group, with a compound of general formula XII

$$HS-(\overset{\overset{\textstyle R^1}{|}}{CH})_n-Q \qquad \text{XII}$$

in which Q, *n* and $R^1$ are as previously defined, or with a reactive derivative thereof, wherein the group $R_5$ is different from the group

$$-(\overset{\overset{\textstyle R^1}{|}}{CH})_n-Q$$

by which it is replaced,

C) intramolecular cyclisation of a compound having the general formula XIII

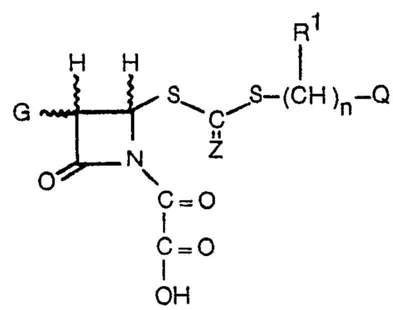

XIII

in which G, n, $R^1$ and Q are as defined above and Z is oxygen or sulphur, in the presence of a trivalent organophosphorous compound;

D) for the preparation of a compound of formula I in which at least one $R^1$ is cyano or fluoro, converting at least one hydroxyl group representing $R^1$ in a compound of formula I into the cyano or fluoro group;

wherein in the processes A, B, C or D, any functional groups are protected if necessary or desired,

the process A, B, C, or D, being followed by removal of any protecting groups, before or after any desired separation of a stereoisomer, and isolation of the resulting penem compound of formula I as the free acid, pharmaceutically acceptable salt or pharmaceutically acceptable ester.

2. A process as defined in Claim 1, characterized in that there is produced a compound of formula I or a

pharmaceutically acceptable salt or ester thereof, in which Q is chosen from pyrrolyl, imidazolyl, pyrazolyl, 1,2,4-triazolyl, 4,1,2-triazolyl, 1,2,3-triazolyl, 2,1,3-triazolyl, 1,2,3,4-tetrazolyl and 2,1,3,4-tetrazolyl, said group Q being unsubstituted or substituted by 1 to 4 R groups as defined in claim 1.

3. A process as defined in claim 1 or 2, characterized in that there is produced a compound of formula I as defined in claim 1 in which R is chosen from hydrogen, amino, hydroxy and loweralkyl, or a pharmaceutically acceptable salt of ester thereof.

4. A process as defined in any one of claims 1 to 3, characterized in that there is produced a compound of formula I as defined in Claim 1, wherein Q is a substituted or unsubstituted imidazol, or a pharmaceutically acceptable salt or ester thereof.

5. A compound as defined in claim 4, characterized in that there is produced a compound of formula 1, as defined in claim 1, wherein Q is unsubstituted imidazolyl, or a pharmaceutically acceptable salt or ester thereof.

6. A process as defined in any one of the preceding claims, characterized in that there is produced a compound of formula 1 as defined in claim 1, wherein n is 2 and $R^1$ is hydrogen or a substituted lower alkyl group as defined in claim 1, or a pharmaceutically acceptable salt or ester thereof.

7. A process as defined in Claim 1, characterized in that there is produced:
5R,6S,8R-2-[2-(imidazol-1-yl)ethylthio]-6-(1-hydroxyethyl]penem-3-carboxylic acid,
5R,6S,8R-2-[2-(1,2,4-1*H*-triazol-1-yl)ethylthio]-6-(hydroxyethyl)-penem-3-carboxylic acid,
5R,6S,8R-2-[2-(1,2,3-triazolyl-1-yl)ethylthio]-6-(1-hydroxyethyl)penem-3-carboxylic acid,
5R,6S,8R-2-[2-(2-methyl-5-nitroimidazol-1-yl)ethylthio]-6-(1-hydroxyethyl)penem-3-carboxylic acid,
5R,6S,8R-2-[2-(tetrazol-1-yl)ethylthio]-6-(1-hydroxyethyl)penem-3-carboxylic acid,
5R,6S,8R-2-[2-(tetrazol-2-yl)ethylthio-6-(1-hydroxyethyl)penem-3-carboxylic acid,
5R,6S,8R-2-[1-(R,S)-methyl-2-(imidazol-1-yl)ethylthio]-6-(1-hydroxyethyl)penem-3-carboxylic acid,
or a pharmaceutically acceptable salt or ester of any one of the foregoing compounds.

8. A process for the preparation of a pharmaceutical composition characterized in that a compound of formula I or a pharmaceutically acceptable salt or ester thereof, as defined in any of the preceding claims, is mixed with a pharmaceutically acceptable carrier or excipient.

9. A process for the preparation of a pharmaceutical composition, characterized in that a compound of formula I or a pharmaceutically acceptable salt or ester thereof prepared by the process of any one of the preceding claims, is mixed with a pharmaceutically acceptable carrier or excipient.

**Patentansprüche für die Vertragsstaaten: BE CH DE FR GB IT LI LU NL SE**

1. Verbindung der Formel

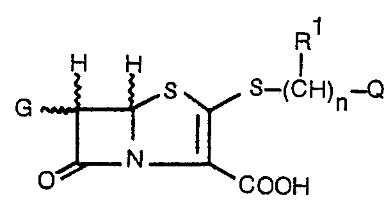

I

worin
G Hydroxyniederalkyl ist,
Q die Gruppe

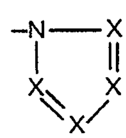

bedeutet, in der jedes X

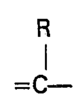

oder =N— ist, mit der Maßgabe, daß zumindest ein X

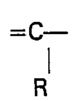 ist;

R ist Wasserstoff, Niederalkyl, Aminoniederalkyl, Mono- und Di-niederalkylamino-niederalkyl, Carboxyniederalkyl, Sulfoniederalkyl, Hydroxyniederalkyl, Cyano, Hydroxy, Amino, Mono- und Di-niederalkylamino, Niederalkylsulfonat, Sulfamyl, Halogen, Hydroxyliminoniederalkyl, Niederalkoxyiminoniederalkyl, Carboxy, Carbamyl, Mono- oder Di-niederalkylcarbamyl, Nitro, Carbamyloxy,

Ureidoniederalkyl oder Carbamylhydrazolniederalkyl; oder zwei R-Gruppen an benachbarten Kohlenstoffatomen bilden zusammen mit den Kohlenstoffatomen, an die sie gebunden sind, einen gemeinsamen aromatischen Ring; jedes $R^1$ ist unabhängig voneinander Wasserstoff, Niederalkyl, Carboxy, Carbamyl, Cyano, Hydroxy, Amino, Niederalkylthio, Fluor, Niederalkoxy, Niederalkanoyloxy, oder Niederalkyl substituiert durch Imidazolyl (welches durch eine durch R definierte Gruppe oder durch Ureido substituiert sein kann) Amino, Niederalkylamino, Carbanoylamino, Ureidoamino, Hydroxy, Cyano, Halogen, Niederalkoxy, Carbamyloxy, Carboxy, Carbamyl, Niederalkylcarbonyl, Sulfo, Sulfamyl, Niederalkylsulfonyl, Niederalkoxysulfonyl, Niederalkoxycarbonyl, Hydroxyniederalkylcarbonyl oder Hydroxyniederalkylsulfonyl, mit der Maßgabe, daß wenn $R_1$ an ein Kohlenstoffatom gebunden ist, das entweder dem in 2-Stellung des Penemringes gebundenen Schwefelatom oder dem Stickstoffatom, über welches die Gruppe Q mit der Seitenkette

$$-(\overset{R^1}{\overset{|}{C}}H)_n-$$

verknüpft ist, benachbart ist, dann kann $R_1$ nicht Hydroxy, Amino oder Fluor sein;

n ist 1 bis 4;

wobei der Ausdruck "nieder" einen Rest mit 1 bis 6 Kohlenstoffatome bezeichnet; und die pharmazeutisch annehmbaren Salze und Ester davon, in racemischer oder optisch aktiver Form.

2. Verbindung nach Anspruch 1, dadurch gekennzeichnet, daß Q ausgewählt ist aus Pyrrolyl, Imidazolyl, Pyrazolyl, 1,2,4-Triazolyl, 4,1,2-Triazolyl, 1,2,3-Triazolyl, 2,1,3-Triazolyl, 1,2,3,4-Tetrazolyl und 2,1,3,4-Tetrazolyl, und die Gruppe Q unsubstituiert oder durch 1 bis 4 R-Gruppen, wie sie in Anspruch 1 definiert sind, substituiert ist.

3. Verbindung nach Anspruch 1 oder 2, dadurch gekennzeichnet, daß R ausgewählt ist aus Wasserstoff, Amino, Hydroxy und Niederalkyl.

4. Verbindung nach einem der Ansprüche 1 bis 3, dadurch gekennzeichnet, daß Q ein substituiertes oder unsubstituiertes Imidazol ist.

5. Verbindung nach Anspruch 4, dadurch gekennzeichnet, daß Q ein unsubstituiertes Imidazol ist.

6. Verbindung nach einem der vorgherhenden Ansprüche, dadurch gekennzeichnet, daß n 2 und $R^1$ Wasserstoff oder eine substituierte Niederalkyl-Gruppe, wie in Anspruch 1 definiert, ist.

7. Verbindung nach Anspruch 1, namlich:

5R,6S,8R-2-[2-(Imidazol-1-yl)ethylthio]-6-(1-hydroxyethyl]penem-3-carbonsäure,

5R,6S,8R-2-[2-(1,2,4-1*H*-Triazol-1-yl)ethylthio]-6-(hydroxyethyl)-penem-3-carbonsäure,

5R,6S,8R-2-[2-(1,2,3-Triazol-1-yl)ëthylthio]-6-(1-hydroxyethyl)penem-3-carbonsäure,

5R,6S,8R-2-[2-(2-Methyl-5-nitroimidazol-1-yl)ethylthio]-6-(1-Hydroxyethyl)penem-3-carbonsäure,

5R,6S,8R-2-[2-(Tetrazol-1-yl)ethylthio]-6-(1-hydroxyethyl)penem-3-carbonsäure,

5R,6S,8R-2-[2-(2-Tetrazol-2-yl)ethylthio-6-(1-hydroxyethyl)penem-3-carbonsäure,

5R,6S,8R-2-[1-(R,S)-Methyl-2-(imidazol-1-yl)ethylthio]-6-(1-hydroxyethyl)penem-3-carbonsäure,

oder ein pharmazeutisch annehmbares Salz oder Ester einer der vorstehenden Verbindungen.

8. Pharmazeutische Zusammensetzung, enthaltend eine Verbindung, wie in einem der vorstehenden Ansprüche definiert, im Gemisch mit einem pharmazeutisch annehmbaren Träger oder Hilfsstoff.

9. Zusammensetzung nach Anspruch 8, dadurch gekennzeichnet, daß die Verbindung 5R,6S,8R-2-[2-(Imidazol-1-yl)-ethylthio)-6-(1-hydroxyethyl)penem-3-carbonsäure oder ein pharmazeutisch annehmbares Salz oder Ester davon, ist.

10. Verfahren zur Herstellung einer Verbindung nach Anspruch 1, gekennzeichnet durch

A) Überführung einer Verbindung der Formel [IX(a), IX(b)]

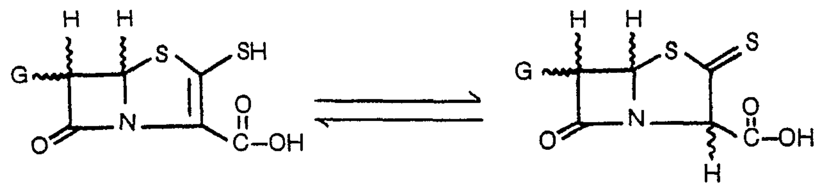

IX(a) IX(b)

in welcher G obige Bedeutung besitzt, in eine Verbindung der Formel I durch Einführung der Gruppe

$$-(\overset{R^1}{\overset{|}{C}}H)_n-Q$$

(in welcher Q, *n* und $R^1$ obige Bedeutung besitzen)

(i) durch Reaktion einer Verbindung der Formel X

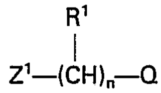

X

in welcher Q, $R^1$ und n obige Bedeutung besitzen, und $Z^1$ eine Abgangsgruppe ist, mit der tautomeren Form der Formel IX(a) und IX(b), oder

(ii) durch Reaktion einer Verbindung der Formel $X^1$

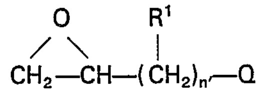

in welcher Q und $R^1$ obige Bedeutung besitzen, und n′ für Null bis 2 steht, mit der tautomeren Form der Formel IX(a) und IX(b);

B) Reaktion einer Verbindung der allgemeinen Formel XI

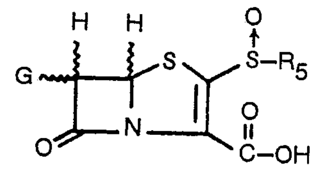

XI

in welcher G obige Bedeutung besitzt und $R_5$ eine organische Gruppe ist, mit einer Verbindung der allgemeinen Formel XII

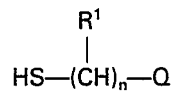

XII

in welcher Q, *n* und $R^1$ obige Bedeutung besitzen, oder mit einem reaktiven Derivat davon, wobei die Gruppe $R_5$ verschieden ist von der Gruppe

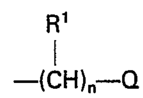

durch welche sie ersetzt wird;

C) intramolekulare Cyclisierung einer Verbindung mit der allgemeinen Formel XIII

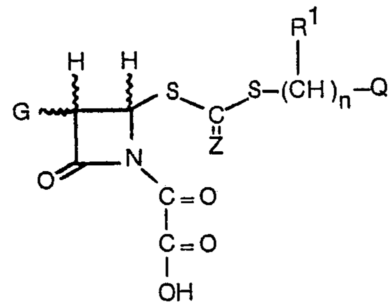

XIII

in welcher G, n, $R^1$ und Q obige Bedeutung besitzen und Z für Sauerstoff oder Schwefel steht, in Gegenwart einer trivalenten Organophosphorverbindung;

D) zur Herstellung einer Verbindung der Formel I, in welcher mindestens ein $R^1$ Cyano oder Fluor ist, Umwandlung mindestens einer den Rest $R^1$ in einer Verbindung der Formel I darstellenden Hydroxylgruppe in die Cyano- oder Fluorgruppe;

wobei in den Verfahren A, B, C oder D funktionelle Gruppen, falls notwendig oder gewünscht, geschützt sind, und anschließend an das Verfahren A, B, C oder D die Entfernung der Schutzgruppen vor oder nach einer erwünschten Abtrennung eines Stereoisomeren erfolgt, und Isolierung der erhaltenen Penem-Verbindung der Formel I als freie Saüre, pharmazeutisch annehmbares Salz oder pharmazeutisch annehmbarer Ester.

**Patentsansprüche fur den Vertragsstaat: AT**

1. Verfahren zur Herstellung einer Verbindung der Formel

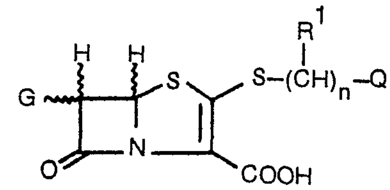

I

worin
G Hydroxyniederalkyl ist,
Q die Gruppe

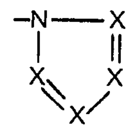

bedeutet, in der jedes X

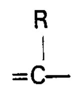

oder =N— ist, mit der Maßgabe, daß zumindest ein X

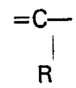 ist;

R ist Wasserstoff, Niederalkyl, Aminoniederalkyl, Mono- und Di-niederalkylamino-niederalkyl, Carboxyniederalkyl, Sulfoniederalkyl, Hydroxyniederalkyl, Cyano, Hydroxy, Amino, Mono- und Di-niederalkylamino, Niederalkylsulfonat, Sulfamyl, Halogen, Hydroxyliminoniederalkyl, Niederalkoxyiminoniederalkyl, Carboxy, Carbamyl, Mono- oder Di-niederalkylcarbamyl, Nitro, Carbamyloxy, Ureidoniederalkyl oder Carbamylhydrazolniederalkyl; oder zwei R-Gruppen an benachbarten Kohlenstoffatomen bilden zusammen mit den Kohlenstoffatomen, an die sie gebunden sind, einen gemeinsamen aromatischen Ring; jedes $R^1$ ist unabhängig voneinander Wasserstoff, Niederalkyl, Carboxy, Carbamyl, Cyano, Hydroxy, Amino, Niederalkylthio, Fluor, Niederalkoxy, Niederalkanoyloxy, oder Niederalkyl substituiert durch Imidazolyl (welches durch eine durch R definierte Gruppe oder durch Ureido substituiert sein kann) Amino, Niederalkylamino, Carbanoylamino, Ureidoamino, Hydroxy, Cyano, Halogen, Niederalkoxy, Carbamyloxy, Carboxy, Carbamyl, Niederalkylcarbonyl, Sulfo, Sulfamyl, Niederalkylsulfonyl, Niederalkoxysulfonyl, Niederalkoxycarbonyl, Hydroxyniederalkylcarbonyl oder Hydroxyniederalkylsulfonyl, mit der Maßgabe, daß wenn $R_1$ an ein Kohlenstoffatom gebunden ist, das entweder dem in 2-Stellung des Penemringes gebundenen Schwefelatom oder dem Stickstoffatom, über welches die Gruppe Q mit der Seitenkette

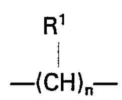

verknüpft ist, benachbart ist, dann kann $R_1$ nicht Hydroxy, Amino oder Fluor sein;

n ist 1 bis 4;

wobei der Ausdruck "nieder" einen Rest mit 1 bis 6 Kohlenstoffatome bezeichnet; und die pharmazeutisch annehmbaren Salze und Ester davon, in racemischer oder optisch aktiver Form.

A) Überführung einer Verbindung der Formel [IX(a), IX(b)]

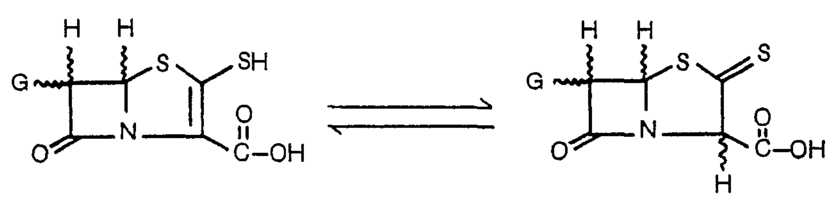

IX(a) IX(b)

in welcher G obige Bedeutung besitzt, in eine Verbindung der Formel I durch Einführung der Gruppe

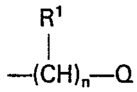

(in welcher Q, *n* und $R^1$ obige Bedeutung besitzen)

(i) durch Reaktion einer Verbindung der Formel X

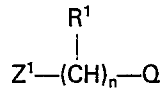

X

in welcher Q, $R^1$ und n obige Bedeutung besitzen, und $Z^1$ eine Abgangsgruppe ist, mit der tautomeren Form der Formel IX(a) und IX(b), oder

(ii) durch Reaktion einer Verbindung der Formel $X^1$

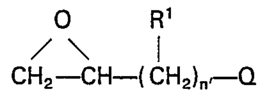

in welcher Q und $R^1$ obige Bedeutung besitzen, und n' für Null bis 2 steht, mit der tautomeren Form der Formel IX(a) und IX(b);

B) Reaktion einer Verbindung der allgemeinen Formel XI

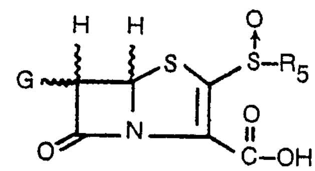

XI

in welcher G obige Bedeutung besitzt und $R_5$ eine organische Gruppe ist, mit einer Verbindung der allgemeinen Formel XII

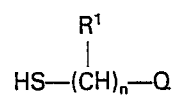

XII

in welcher Q, *n* und $R^1$ obige Bedeutung besitzen, oder mit einem reaktiven Derivat davon, wobei die Gruppe $R_5$ verschieden ist von der Gruppe

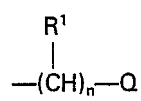

durch welche sie ersetzt wird;

C) intramolekulare Cyclisierung einer Verbindung mit der allgemeinen Formel XIII

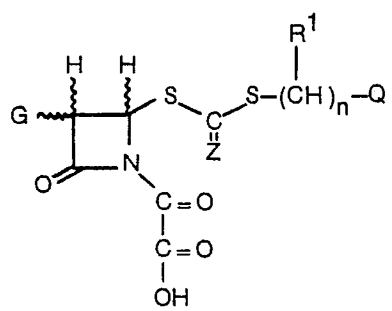

XIII

in welcher G, n, $R^1$ und Q obige Bedeutung besitzen und Z für Sauerstoff oder Schwefel steht, in Gegenwart einer trivalenten Organophosphorverbindung;

D) zur Herstellung einer Verbindung der Formel I, in welcher mindestens ein $R^1$ Cyano oder Fluor ist, Umwandlung mindestens einer den Rest $R^1$ in einer Verbindung der Formel I darstellenden Hydroxylgruppe in die Cyano- oder Fluorgruppe;

wobei in den Verfahren A, B, C oder D funktionelle Gruppen, falls notwendig oder gewünscht, geschützt sind, und anschließend an das Verfahren A, B, C oder D die Entfernung der Schutzgruppen vor

oder nach einer erwünschten Abtrennung eines Stereoisomeren erfolgt, und Isolierung der erhaltenen Penem-Verbindung der Formel I als freie Saüre, pharmazeutisch annehmbares Salz oder pharmazeutisch annehmbarer Ester.

2. Verfahren nach Anspruch 1, dadurch gekennzeichnet, daß eine Verbindung der Formel I oder ein pharmazeutisch annehmbares Salz oder Ester davon hergestellt wird, in der Q ausgewählt aus Pyrrolyl, Imidazolyl, Pyrazolyl, 1,2,4-Triazolyl, 4,1,2-Triazolyl, 1,2,3-Triazolyl, 2,1,3-Triazolyl, 1,2,3,4-Tetrazolyl und 2,1,3,4-Tetrazolyl, und die Gruppe Q unsubstituiert oder durch 1 bis 4 R-Gruppen, wie sie in Anspruch 1 definiert sind, substituiert ist.

3. Verfahren nach Anspruch 1 oder 2, dadurch gekenzeichnet, daß eine Verbindung der im Anspruch 1 definierten Formel I hergestellt wird, in der R ausgewählt ist aus Wasserstoff, Amino, Hydroxy und Niederalkyl, oder ein pharmazeutisch annehmbares Salz oder Ester davon.

4. Verfahren nach einem der Ansprüche 1 bis 3, dadurch gekennzeichnet, daß eine Verbindung der in Anspruch 1 definierten Formel I hergestellt wird, worin Q ein substituiertes oder unsubstituiertes Imidazol ist, oder ein pharmazeutisch annehmbares Salz oder Ester davon.

5. Eine Verbindung nach Anspruch 4, dadurch gekennzeichnet, daß eine Verbindung der in Anspruch 1 definierten Formel I hergestellt wird, worin Q ein unsubstituiertes Imidazolyl ist, oder ein pharmazeutisch annnehmbares Salz oder Ester davon.

6. Verfahren nach einem der vorhergehenden Ansprüche, dadurch gekennzeichnet, daß eine Verbindung der in Anspruch 1 definierten Formel I hergestellt wird, worin n 2 und $R^1$ Wasserstoff oder eine substituierte Niederalkyl-Gruppe, wie in Anspruch 1 definiert, ist, oder ein pharmazeutisch annehmbares Salz oder Ester davon.

7. Verfahren nach Anspruch 1, dadurch gekennzeichnet, daß hergestellt wird:
5R,6S,8R-2-[2-(Imidazol-1-yl)ethylthio]-6-(1-hydroxyethyl]penem-3-carbonsäure,
5R,6S,8R-2-[2-(1,2,4-1*H*-Triazol-1-yl)ethylthio]-6-(hydroxyethyl)-penem-3-carbonsäure,
5R,6S,8R-2-[2-(1,2,3-Triazol-1-yl)ethylthio]-6-(1-hydroxyethyl)penem-3-carbonsäure,
5R,6S,8R-2-[2-(2-Methyl-5-nitroimidazol-1-yl)ethylthio]-6-(1-Hydroxyethyl)penem-3-carbonsäure,
5R,6S,8R-2-[2-(Tetrazol-1-yl)ethylthio]-6-(1-hydroxyethyl)penem-3-carbonsäure,
5R,6S,8R-2-[2-(2-Tetrazol-2-yl)ethylthio-6-(1-hydroxyethyl)penem-3-carbonsäure,
5R,6S,8R-2-[1-(R,S)-Methyl-2-(imidazol-1-yl)ethylthio]-6-(1-hydroxyethyl)penem-3-carbonsäure,
oder ein pharmazeutisch annehmbares Salz oder Ester einer der vorstehenden Verbindungen.

8. Verfahren zur Herstellung einer pharmazeutischen Zusammensetzung, dadurch gekennzeichnet, daß eine Verbindung der Formel I oder ein pharmazeutisch annehmbares Salz oder Ester davon, wie in einem der vorstehenden Ansprüche definiert, mit einem pharmazeutisch annehmbaren Träger oder Hilfsstoff vermischt wird.

9. Verfahren zur Herstellung einer pharmazeutischen Zusammensetzung, dadurch gekennzeichnet, daß eine Verbindung der Formel I oder ein pharmazeutisch annehmbares Salz oder Ester davon, hergestellt durch ein Verfahren nch einem der vorgehenden Ansprüche, mit einem pharmazeutisch annehmbaren Träger oder Hilfsstoff vermischt wird.

**Revendications pour les Etats contractants: BE CH DE FR GB IT LI LU NL SE**

1. Un composé de formule

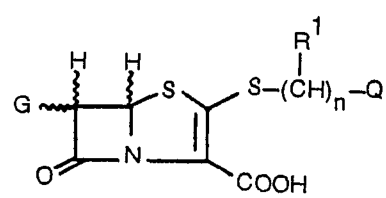

I

dans laquelle

G est un hydroxyalcoyle inférieur;

Q est le groupe

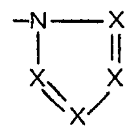

dans lequel chaque X est

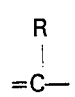

ou =N—, à condition qu'au moins un X soit

$$=\underset{\displaystyle R}{\underset{|}{C}}—;$$

R est hydrogène, alcoyle inférieur, amino-alcoyle inférieur, mono- et di-alkyl inférieur-amino-alcoyle inférieur, carboxy-alcoyle inférieur, sulfo-alcoyle inférieur, hydroxy-alcoyle inférieur, cyano, hydroxy, amino, mono- et di-alkyl-amino inférieur, alkyl inférieur-sulfonate, sulfamyle, halogéno, hydroxylimino-alcoyle inférieur, alcoxy inférieur-imino-alcoyle inférieur, carboxy, carbamyle, mono- ou di-alkyl inférieur-carbamyle, nitro, carbamyloxy, uréido-alcoyle inférieur ou carbamylhydrazo-alcoyle inférieur; ou deux groupes R attachés à des atomes de carbone adjacents, avec les atomes auxquels ils sont attachés, forment un noyau aromatique fusionné;

chaque $R^1$ est indépendamment hydrogène, alcoyle inférieur, carboxy, carbamyle, cyano, hydroxy, amino, alkylthio inférieur, fluoro, alcoxy inférieur, alcanoyloxy inférieur, ou alcoyle inférieur, substitué par imidazolyle (qui peut être substitué par un groupe défini par R ou par uréido), amino, alkyl inférieur-amino, carbamoylamino, uréidoamino, hydroxy, cyano, halogène, alcoxy inférieur, carbamyloxy, carboxy, carbamyle, alkyl inférieur-carbonyle, sulfo, sulfamyle, alkylsulfonyle inférieur, alcoxysulfonyle inférieur, alcoxycarbonyle inférieur, hydroxy-alkyl inférieur-carbonyle ou hydroxy-alkyl inférieur-sulfonyle, à condition que lorsque $R_1$ est attaché à un atome de carbone adjacent soit à l'atome de soufre connecté à la position 2 du noyau pénem ou à l'atome d'azote par lequel le groupe Q est connecté à la chaîne latérale

$$—(\overset{\displaystyle R^1}{\overset{|}{C}}H)_n—$$

alors ce $R^1$ ne puisse être hydroxy, amino ou fluoro;

n est 1 à 4;

et où le terme "inférieur" désigne un radical ayant 1 à 6 atomes de carbone;

et leurs sels et esters acceptables en pharmacie, sous forme racémique ou optiquement active.

2. Composé tel que défini à la revendication 1, caractérisé en ce que Q est choisi parmi pyrrolyle, imidazolyle, pyrazolyle, 1,2,4-triazolyle, 4,1,2-triazolyle, 1,2,3-triazolyle, 2,1,3-triazolyle, 1,2,3,4-tétrazolyle et 2,1,3,4-tétrazolyle, ledit groupe Q étant non substitué ou substitué par 1 à 4 groupes R tels que définis à la revendication 1.

3. Composé tel que défini à la revendication 1 ou 2, caractérisé en ce que R est choisi parmi hydrogène, amino, hydroxy et alcoyle inférieur.

4. Composé tel que défini selon l'une des revendications 1 à 3, caractérisé en ce que Q est un imidazolyle substitué ou non substitué.

5. Composé tel que défini à la revendication 4, caractérisé en ce que Q est un imidazolyle non substitué.

6. Composé tel que défini selon l'une quelconque des revendications précédentes, caractérisé en ce que n est 2 et $R^1$ est hydrogène ou un groupe alcoyle inférieur substitué tel que défini à la revendication 1.

7. Composé tel que défini à la revendication 1, c'est-à-dire:

acide 5R,6S,8R-2-[2-(imidazol-1-yl)éthylthio]-6-(1-hydroxyéthyl]pénem-3-carboxylique,

acide 5R,6S,8R-2-[2-(1,2,4-1*H*-triazol-1-yl)éthylthio]-6-(1-hydroxyéthyl)-pénem-3-carboxylique,

acide 5R,6S,8R-2-[2-(1,2,3-triazol-1-yl)éthylthio]-6-(1-hydroxyéthyl)pénem-3-carboxylique,

acide 5R,6S,8R-2-[2-(2-méthyl-5-nitroimidazol-1-yl)éthylthio]-6-(1-hydroxyéthyl)pénem-3-carboxylique,

acide 5R,6S,8R-2-[2-(tétrazol-1-yl)éthylthio]-6-(1-hydroxyéthyl)pénem-3-carboxylique,

acide 5R,6S,8R-2-[2-(tétrazol-2-yl)éthylthio-6-(1-hydroxyéthyl)pénem-3-carboxylique,

acide 5R,6S,8R-2-[1-(R,S)-méthyl-2-(imidazol-1-yl)éthylthio]-6-(1-hydroxyéthyl)pénem-3-carboxylique,

ou un sel ou ester acceptable en pharmacie de l'un des composés ci-dessus.

8. Composition pharmaceutique comprenant un composé tel que défini selon l'une des revendications précédentes en mélange avec un véhicule ou excipient acceptable en pharmacie.

9. Composition telle que définie à la revendication 8, caractérisé en ce que le composé est l'acide 5R,6S,8R-2-[2-(imidazol-1-yl)-éthylthio)-6-(1-hydroxyéthyl)pénem-3-carboxylique ou son sel ou ester acceptable en pharmacie.

10. Procédé pour la préparation d'un composé tel que défini à la revendication 1, caractérisé en ce que

A) un composé de formule [IX(a), IX(b)]

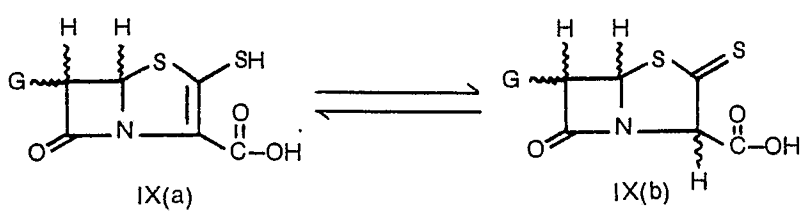

IX(a) IX(b)

où G est tel que défini ci-dessus, est transformé en un composé de formule I par introduction du groupe

$$-\overset{R^1}{\overset{|}{(CH)}}_n-Q$$

(où Q, *n* et $R^1$ sont tels que précédemment définis) par

(i) réaction d'un composé de formule X

$$Z^1-\overset{R^1}{\overset{|}{(CH)}}_n-Q \qquad X$$

dans laquelle Q, $R^1$ et n sont tels que définis ci-dessus et $Z^1$ est un groupe partant avec le tautomère de formule IX(a) et IX(b), ou (ii) la réaction d'un composé de formule $X^1$

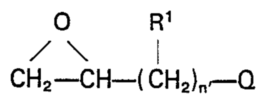

dans laquelle Q et $R^1$ sont tels que définis ci-dessus et n' est zéro à 2, avec le tautomère des formules IX(a) et IX(b)

B) La réaction d'un composé de formule générale XI

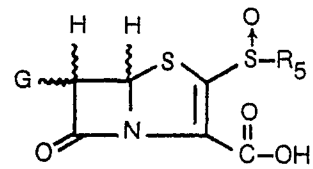

XI

dans laquelle G est tel que défini ci-dessus et $R_5$ est un groupe organique, avec un composé de formule générale XII

$$HS-\overset{R^1}{\overset{|}{(CH)}}_n-Q \qquad XII$$

dans laquelle Q, *n* et $R^1$ sont tels que précédemment définis ou avec un dérivé réactif, où le groupe $R_5$ est différent du groupe

$$-\overset{R^1}{\overset{|}{(CH)}}_n-Q$$

par lequel il est remplacé,

C) cyclisation intramoléculaire d'un composé ayant la formule générale XIII

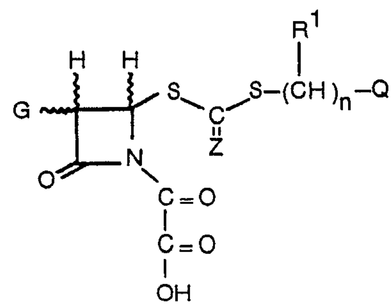

XIII

dans laquelle G, n, $R^1$ et Q sont tels que définis ci-dessus et Z est oxygène ou soufre, en présence d'un composé organophosphoreux trivalent;

D) pour la préparation d'un composé de formule I dans laquelle au moins l'un de $R^1$ est cyano ou fluoro, la conversion d'au moins un groupe hydroxyle représentant $R^1$ en un composé de formule I en le groupe cyano ou fluoro;

où, dans les procédés A, B, C ou D, tous les groupes fonctionnels sont protégés si nécessaire ou souhaité, le procédé A, B, C, ou D étant suivi de l'enlèvement de tout groupe protecteur, avant ou après toute séparation souhaitée d'un stéréoisomère et isolement du composé pénème résultant de la formule I sous la forme de l'acide libre, du sel acceptable en pharmacie ou de l'ester acceptable en pharmacie.

**Revendications pour l'Etat contractant: AT**

1. Procédé pour la préparation d'un composé de formule

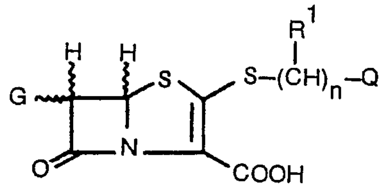

I

dans laquelle

G est un hydroxyalcoyle inférieur;

Q est le groupe

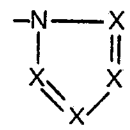

dans lequel chaque X est

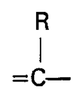

ou =N—, à condition qu'au moins un X soit

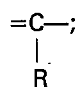

R est hydrogène, alcoyle inférieur, amino-alcoyle inférieur, mono- et di-alkyl inférieur-amino-alcoyle inférieur, carboxy-alcoyle inférieur, sulfo-alcoyle inférieur, hydroxy-alcoyle inférieur, cyano, hydroxy, amino, mono- et di-alkyl-amino inférieur, alkyl inférieur-sulfonate, sulfamyle, halogéno, hydroxylimino-alcoyle inférieur, alcoxy inférieur-imino-alcoyle inférieur, carboxy, carbamyle, mono- ou di-alkyl inférieur-carbamyle, nitro, carbamyloxy, uréido-alcoyle inférieur ou carbamylhydrazo-alcoyle inférieur; ou deux groupes R attachés à des atomes de carbone adjacents, avec les atomes auxquels ils sont attachés, forment un noyau aromatique fusionné;

chaque $R^1$ est indépendamment hydrogène, alcoyle inférieur, carboxy, carbamyle, cyano, hydroxy, amino, alkylthio inférieur, fluoro, alcoxy inférieur, alcanoyloxy inférieur, ou alcoyle inférieur substitué par imidazolyle (qui peut être substitué par un groupe défini par R ou par uréido), amino, alkyl inférieur-amino, carbamoylamino, uréidoamino, hydroxy, cyano, halogène, alcoxy inférieur, carbamyloxy, carboxy, carbamyle, alkyl inférieur-carbonyle, sulfo, sulfamyle, alkylsulfonyle inférieur, alcoxysulfonyle inférieur, alcoxycarbonyle inférieur, hydroxy-alkyl inférieur-carbonyle ou hydroxy-alkyl inférieur-sulfonyle, à condition que lorsque $R_1$ est attaché à un atome de carbone adjacent soit à l'atome de soufre connecté à la position 2 du noyau pénem ou à l'atome d'azote par lequel le groupe Q est connecté à la chaîne latérale

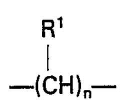

alors ce $R^1$ ne puisse être hydroxy, amino ou fluoro;

n est 1 à 4;

et où le terme "inférieur" désigne un radical ayant 1 à 6 atomes de carbone;

et leurs sels et esters acceptables en pharmacie, sous forme racémique ou optiquement active.

caractérisé en ce que:

A) un composé de formule [IX(a), IX(b)]

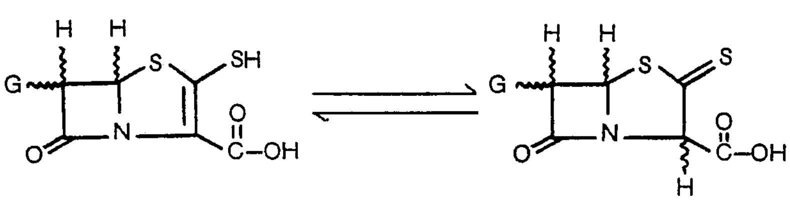

IX(a) IX(b)

où G est tel que défini ci-dessus, est transformé en un composé de formule I par introduction du groupe

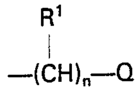

(où Q, *n* et $R^1$ sont tels que précédemment définis) par

(i) réaction d'un composé de formule X

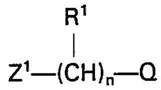

X

dans laquelle Q, $R^1$ et n sont tels que définis ci-dessus et $Z^1$ est un groupe partant avec le tautomère de formule IX(a) et IX(b), ou (ii) la réaction d'un composé de formule $X^1$

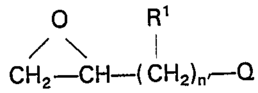

dans laquelle Q et $R^1$ sont tels que définis ci-dessus et n' est zéro à 2, avec le tautomère des formules IX(a) et IX(b).

B) La réaction d'un composé de formule générale XI

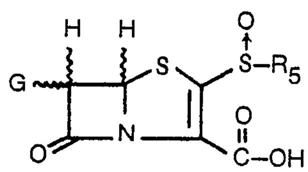

XI

dans laquelle G est tel que défini ci-dessus et $R_5$ est un groupe organique, avec un composé de formule générale XII

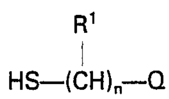

XII

dans laquelle Q, *n* et $R^1$ sont tels que précédemment définis, ou avec un dérivé réactif, où le groupe $R_5$ est différent du groupe

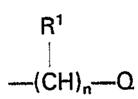

par lequel il est remplacé.

C) cyclisation intramoléculaire d'un composé ayant la formule générale XIII

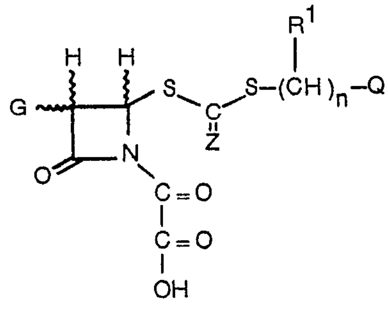

XIII

dans laquelle G, n, $R^1$ et Q sont tels que définis ci-dessus et Z est oxygène ou soufre, en présence d'un composé organophosphoreux trivalent;

D) pour la préparation d'un composé de formule I dans laquelle au moins un $R^1$ est cyano ou fluoro, la conversion d'au moins un groupe hydroxyle représentant $R^1$ en un composé de formule I en le groupe cyano ou fluoro;

où, dans les procédés A, B, C ou D, tous les groupes fonctionnels sont protégés si nécessaire ou souhaité, le procédé A, B, C ou D étant suivi de l'enlèvement de tout groupe protecteur, avant ou après toute séparation souhaitée d'un stéréoisomère et isolement du composé pénème résultant de la formule I sous la forme de l'acide libre, du sel acceptable en pharmacie ou de l'ester acceptable en pharmacie.

2. Procédé selon la revendication 1, caractérisé en ce qu'on produit un composé de formule I, ou son sel ou ester acceptable en pharmacie, où Q est choisi parmi pyrrolye, imidazolyle, pyrazolyle, 1,2,4-triazolyle, 4,1,2-triazolyle, 1,2,3-triazolyle, 2,1,3-triazolyle, 1,2,3,4-tétrazolyle et 2,1,3,4-tétrazolyle, ledit groupe Q étant non substitué ou substitué par 1 à 4 groupes R tels que définis à la revendication 1.

3. Procédé tel que défini à la revendication 1 ou 2, caractérisé en ce qu'on produit un composé de formule I, tel que défini à la revendication 1, dans lequel R est choisi parmi hydrogène, amino, hydroxy et alcoyle inférieur, ou son sel ou ester acceptable en pharmacie.

4. Procédé tel que défini selon l'une des revendications 1 à 3, caractérisé en ce que qu'on produit un composé de formule I tel que défini à la revendication 1, où Q est un imidazole substitué ou non substitué, ou son sel ou ester acceptable en pharmacie.

5. Composé tel que défini à la revendication 4, caractérisé en ce qu'on produit un composé de formule 1, tel que défini à la revendication 1, où Q est imidazolyle non substitué, ou son sel ou ester acceptable en pharmacie.

6. Procédé selon l'une quelconque des revendications précédentes, caractérisé en ce qu'on produit un composé de formule I, tel que défini à la revendication 1 où n est 2 et $R^1$ est hydrogène ou un groupe alcoyle inférieur substitué tel que défini à la revendication 1 ou son sel ou ester acceptable en pharmacie.

7. Procédé tel que défini à la revendication 1, caractérisé en ce qu'on produit:

acide 5R,6S,8R-2-[2-(imidazol-1-yl)éthylthio]-6-(1-hydroxyéthyl]pénem-3-carboxylique,

acide 5R,6S,8R-2-[2-(1,2,4-1*H*-triazol-1-yl)éthylthio]-6-(1-hydroxyéthyl)-pénem-3-carboxylique,

acide 5R,6S,8R-2-[2-(1,2,3-triazol-1-yl)éthylthio]-6-(1-hydroxyéthyl)pénem-3-carboxylique,

acide 5R,6S,8R-2-[2-(2-méthyl-5-nitroimidazol-1-yl)éthylthio]-6-(1-hydroxyéthyl)pénem-3-carboxylique,

acide 5R,6S,8R-2-[2-(tétrazol-1-yl)éthylthio]-6-(1-hydroxyéthyl)pénem-3-carboxylique,

acide 5R,6S,8R-2-[2-(tétrazol-2-yl)éthylthio-6-(1-hydroxyéthyl)pénem-3-carboxylique,

acide 5R,6S,8R-2-[1-(R,S)-méthyl-2-(imidazol-1-yl)éthylthio]-6-(1-hydroxyéthyl)pénem-3-carboxylique,

ou un sel ou ester acceptable en pharmacie de l'un des composés qui précèdent.

8. Procédé pour la préparation d'une composition pharmaceutique, caractérisé en ce qu'un composé de formule I ou son sel ou ester acceptable en pharmacie tel que défini selon l'une des revendications précédentes est mélange à un véhicule ou excipient acceptable en pharmacie.

9. Procédé pour la préparation d'une composition pharmaceutique, caractérisé en ce qu'on composé de formule I ou son sel ou ester acceptable en pharmacie préparé par le procédé selon l'une des revendications précédentes est mélangé à un véhicule ou excipient acceptable en pharmacie.